# EUROPEAN PATENT SPECIFICATION

(11) **EP 0 645 397 B1**
(45) Date of publication and mention of the grant of the patent: **09.09.1998**
(21) Application number: 93307676.2
(22) Date of filing: 28.09.1993
(51) Int. Cl.: C07K 5/037, C07K 5/093, C07K 5/113, C12N 9/10, G01N 33/573

(54) **Glutathione analogs and paralog panels comprising glutathione mimics**
Glutathion analoge und paraloge Serien aus Glutathion imitierenden Stoffen
Analogues de glutathione et séries paralogues contenantes des imitateurs de glutathione

(43) Date of publication of application: 29.03.1995
(73) Proprietor: TERRAPIN TECHNOLOGIES, INC., San Francisco, CA 94080 (US)
(72) Inventor: Kauvar, Lawrence, South San Francisco, California 94080 (US); Lyttle, Matthew, Point Reyes Station, California 94956 (US)
(74) Representative: Goldin, Douglas Michael

(56) References cited:
- WO-A-92/19767
- INTERNATIONAL JOURNAL OF PEPTIDE AND PROTEIN RESEARCH vol. 35, no. 1 , January 1990 , COPENHAGEN DK pages 55 - 62 L. SHEH ET AL. 'Synthesis of cyclic peptide homologs of glutathione as potential antitumor agents'
- EMZYME vol. 41, no. 3 , 1989 pages 175 - 180 G.B. PRINCIPATO ET AL. 'Effects of Some S-Blocke Glutathione Derivatives on the Prevalent Glyoxalase II (a Form) of Rat Liver'
- BIOCHEMICAL JOURNAL vol. 269, no. 1 , 1 July 1990 , LONDON UK pages 47 - 54 A.E.P. ADANG ET AL. 'The glutathione-binding site in glutathione S-transferase'
- DATABASE WPI Section Ch, Week 8635, Derwent Publications Ltd., London, GB; Class B04, AN 86-230384 & JP-A-61 162 175 (SATO PHARM KK) 22 July 1986
- PEPTIDE RESEARCH, vol.5, no.6, 1992, page 337 - 342, LYTTLE M.H. ET AL. 'CONSTRUCTION OF AFFINITY SORBENTS UTILIZING GLUTATHIONE ANALOGS'
- BIOCHEM. J., vol.292, no., 1993, page 371 - 377, CASTRO V.M. ET AL. 'GLUATHIONE ANALOGUE SELECTIVELY BIND GLUTATHIONE S-TRANSFERASE ISOENZYMES'
- Book no. , 1993, 'STRUCTURE AND FUNCTION FOR GLUTATHIONE TRANSFERASE, CHAPTER OON PHARMACEUTICAL TARGETING OF GST ISOENZYMESKAUVAR LAWRENCE M.', TEW. ET AL., CRC PRESS, LONDON
- J.CHEM.SOCIETY (C) ORG, vol., no., 1968, page 1219 - 1224, GAMBLE R. ET AL. 'AMINO-ACIDS AND PEPTIDES. PART XXIX. THE USE OF S-BENZYLTHIOMETHYL-L-CYSTEINE IN PEPTIDE SYNTHESIS: SYNTHESIS OF GLUTATHIONE AND HOMOGLUTATHIONE'
- PHYTOCHEMISTRY, vol.25, no.3, 1986, page 679 - 682, KASAI TAKANORI ET AL. 'GAMMA-GLUTAMYL PEPTIDES OF VIGNA RADIATA SEEDS'

## Description

### Technical Field

The invention relates to tripeptide compounds which are novel analogs of glutathione. The invention also concerns panels of tripeptides that are glutathione analogs which have diverse properties and are useful for characterizing glutathione transferases (GSTs) and as solution phase inhibitors of GST.

### Background Art

Glutathione (GSH), in its reduced form, is a tripeptide of the formula: γ-Glu-Cys-Gly. Reduced glutathione has a central role in maintaining the redox condition in cells and is also an essential substrate for glutathione S-transferase (GST) which facilitates the detoxification of foreign substances by a number of mechanisms, including catalysis of the coupling of an electrophilic portion of a toxin, for instance, to glutathione, rendering the toxin more susceptible to clearance. A second mechanism, which also involves glutathione as substrate, resides in the reduction of peroxides with the concomitant oxidation of glutathione.

Adang, A.E.P., et al., Biochem J (1990) 269:47-54, described tripeptide analogs of GSH which interact with various GST isoenzymes at different concentrations. These analogs are modified forms of GSH in which at least one of the glycine, cysteine, or gamma-glutamine residues is replaced by an alternate amino acid residue.

Additional modified forms have been disclosed, for example, by Principato, G.B., et al., Enzyme (1989) 41:175-180, who studied the effect of a tripeptide GSH analog on glyoxalase II enzyme of rat liver. The tripeptide used by this group was of the formula γ-Glu-ρ-chlorophenylcarbonylmethyl-Cys-Ser. Morris, D., in Biochem J (1960) 76:349-353, described the synthesis of γ-Glu-benzyl-Cys-Val. A large number of GSH tripeptide analogs containing a substitution for only one of the three GSH amino acids have been reported and are commercially available.

WO-A-92/19767 refers to methods for selecting therapeutic modulators which affect, preferentially, the glutathione-S-transferase (GST) complement of normal or unwanted cells or tissues.

Sheh et *al, Int. J. Peptide Protein Res.* **35**:55-62 (1990) refers to the synthesis of two cyclic peptide homologues of glutathione, namely cyclo (Glu [Cys-β-Ala-]-OH) and cyclo (Glu [Cys-Gaba-]-OH).

The invention described hereinbelow concerns novel glutathione tripeptide analogs which are useful as affinity ligands on chromatographic supports and as members of panels which are used to characterize the various isoenzymes of glutathione-S-transferase. Glutathione-S-transferases (GSTs) are present in the form of a number of isoenzymes which differ in specific binding abilities, in substrate and inhibitor specificities and in tissue distribution. Particular complements of GST isoenzymes, with their accompanying differences in properties, thus are characteristic of specific tissues or cell types, such as tumor tissues. As GST is central to the overall metabolism of the tissue or cell as it relates to its defense against toxic substances, the character of the complement of the GST for the cell or tissue is important in designing strategies either for the destruction of the cell or tissue, as would be desirable for tumor cells, or for enhancement of its metabolic function, as would be the case for normal tissue.

The various GST isoenzymes are dimeric proteins formed by binary combinations of monomers encoded by at least fifteen known genes in four gene families resulting in the theoretical possibility of several dozen different dimers, even allowing for preferential dimerization of monomers from the same gene family. In addition to the variability that arises from these combinatorial possibilities, the GST isoenzyme subunits are polymorphic in the human population and have been considered to be subject to additional variation due to gene conversion events among the tandemly repeated members of the family. Posttranslational modifications add further to this variability. As each cell or tissue may contain one or several of these theoretically possible enzymes, determination of the GST complement is of great importance.

The present invention, by providing novel glutathione analogs which are useful as sorbents and as solution phase inhibitors, as well as panels that include them, offers improved methods to characterize and manipulate individual GST enzymes or sets thereof.

### Disclosure of the Invention

The invention is directed to reagents useful in characterizing glutathione S-transferase isoenzymes, in determining the GST complements of cells and tissues, and in therapeutic applications. The invention compounds are systematically modified forms of reduced glutathione and panels comprising such analogs having diverse properties with respect to the targeted enzymes. The invention compounds are also useful as chromatographic affinity ligands, binding reagents, and enzyme inhibitors.

The esters of the tripeptides of the invention are particularly useful in therapeutic and diagnostic contexts; the amides are also capable of derivatization to moieties that can modify pharmacological properties or physical behavior. Thus, in one aspect,- the invention is directed to the alkyl (1-6C) arylalkyl (7-12C) esters and amides of a compound of the formula: 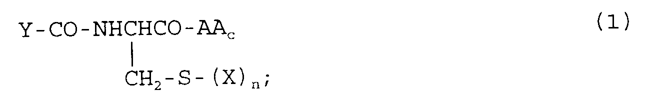
wherein: n is 1 or 2;
wherein when n is 1, X is a mono- or disubstituted or unsubstituted hydrocarbyl (1-20C) moiety optionally containing 1 or 2 nonadjacent heteroatoms (O, S or N), and wherein said substitution is selected from the group consisting of halo, -NO, -NO₂, -NR₂, OR, and SR, wherein R is H or lower alkyl (1-4C);
when n is 2, one X is as above-defined and the other X is lower alkyl (1-4C);
Y is selected from the group consisting of 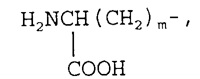 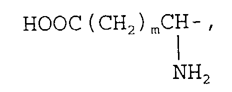 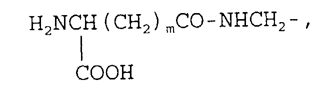 and 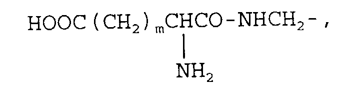
   wherein m is 1 or 2; and
   AA_{c} is an amino acid, coupled through a peptide bond to the remainder of the compound of formula 1, other than β-alanine or glycine.

In another aspect, the invention is direct to a cycloamido form of a compound of the formula 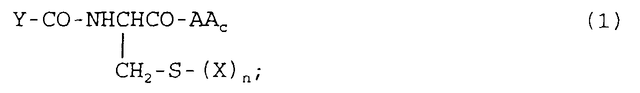 or the alkyl (1-6C) or arylalkyl (7-12C) esters or amides, or salts thereof,
wherein said cycloamido linkage is formed from an amino group of Y and a carboxyl group of AA_{c} or from a carboxyl group of Y and a free amino group of AA_{c};
wherein n is 1 or 2;
wherein when n is 1, X is H or is a mono- or disubstituted or unsubstituted hydrocarbyl (1-20C) moiety optionally containing 1 or 2 nonadjacent heteroatoms (O, S or N), and wherein said substitution is selected from the group consisting of halo, -NO, -NO₂, -NR₂, OR, and SR, wherein R is H or lower alkyl (1-4C); when n is 2, one X is as above defined and the other X is lower alkyl (1-4C);
Y is selected from the group consisting of 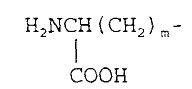 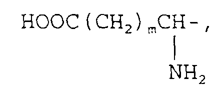 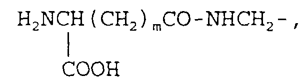 and 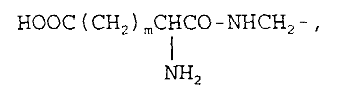
   wherein m is 1 or 2; and
   AA_{c} is valine, alanine, phenylglycine, histidine, tryptophan, tyrosine, phenylalanine or substituted phenlyalanine coupled through a peptide bond to the remainder of formula 1.

In still another aspect, the invention is directed to a method to purify or characterize a human glutathione S-transferase (GST) enzyme which comprises contacting a sample thought to contain the enzyme with a solid support to which is coupled a compound of formula 1, as defined above, under conditions wherein the human GST is adsorbed to the solid support, separating the support from the sample and eluting the human GST from the support.

In still other aspects, the invention is directed to methods to detect the presence or absence of a GST enzyme in the sample, and optionally to characterize it by class, which comprises treating the sample with a compound of formula 1 as defined above and detecting the presence or absence of a complex between any GST contained in the sample and the compound of formula 1.

The invention also includes panels comprising at least five diverse tripeptide glutathione analogs of formula 1 (or the esters, salts, amides or cycloamido forms thereof) wherein the compounds in the panel have diverse characteristics. These panels may be constituted as chromatographic support panels and used to characterize a GST complement of cells.

Finally, the invention is directed to compounds of formula 1 wherein X is a mono-, di-, or trisubstituted aryl (1-20C), preferably benzyl, wherein the substitution is selected from the group consisting of R', halo, -NO, -NO₂, -NH₂, OR, and SR, wherein R is H or alkyl (1-6C), and R' is alkyl (1-6C), alkenyl (1-6C) or alkynyl (1-6C).

### Modes of Carrying Out the Invention

The invention is directed to compounds that are useful individually as chromatographic ligands and as GST inhibitors, as well as to panels of related compounds which have diverse properties and which are useful in determining profiles, for example, of GST enzymes, and in determining GST complement in unknown samples. The invention compounds are systematically modified forms of reduced glutathione and panels comprising such analogs having diverse properties with respect to the targeted enzymes.

When tested by affinity chromatography several of these diverse sorbents selectively bind mammalian GST isoenzymes of a particular class or classes according to the following classification scheme proposed by Mannervik, B., et al., Proc Natl Acad Sci (1985) 82:7202-7206: Alpha (e.g., human α and A1-1, rat 1-1 and 2-2), Mu (human µ, M1a-1a, rat 3-3 and 4-4), Pi (human π and P1-1, rat 7-7) and, a more recently proposed class, Theta (rat 5-5). When tested for inhibition of GST enzymatic activity, several sorbents also act as selective or specific inhibitors of GST isoenzyme classes. Therefore, the compounds, panels and methods of this invention enable novel strategies for the design of drugs targeting cells with elevated levels of particular GST isoenzymes.

### The Compounds of the Invention

The novel compounds of the invention include the moiety of formula 1, wherein one X is a mono- or disubstituted or unsubstituted hydrocarbyl (1-20C) moiety optionally containing one or two nonadjacent heteroatoms (O, S or N), and the other is not present or is lower alkyl (1-4C). In preferred embodiments n is 1 and X is unsubstituted hydrocarbyl. Preferred embodiments for X include methyl, propyl, hexyl, octyl, benzyl and trityl. In the cycloamido forms, X may also be H.

As used herein, "hydrocarbyl" refers to a straight or branched chain or cyclic, saturated or unsaturated, aliphatic or aromatic hydrocarbyl residue containing 1-20C. In addition to the 1-20C, where structurally realistic, the hydrocarbyl may also contain one or two nonadjacent heteroatoms which are O, S or N. Thus, the hydrocarbyl group so modified may be an ether, a diether, a thioether or a dithioether, or a secondary or tertiary amine or diamine. Representative of such substituents include methyl, ethyl, propyl, isopropyl, butyl, tertiary butyl, hexyl, octyl, nonyl, 2,3-dimethyloctyl, dodecyl, 9,9-dimethylundecyl, allyl, 2-butenyl, isobutenyl, cyclohexyl, cyclopentyl, cycloheptyl, phenyl, benzyl, 4-methylbenzyl, triphenylmethyl, methoxyethyl, ethylthioethyl, diethylaminopropyl and the like.

In addition, the hydrocarbyl or hydrocarbyl containing one or two heteroatoms may optionally be substituted by one or two substituents. These substituents may be halo, i.e., fluoro, chloro, bromo or iodo, or hydroxy or sulfhydryl, and/or alkyloxy or alkylthio, such as methylthio, butylthio, propoxy or ethoxy, and/or -NO, -NO₂, or -NH₂, -NH(alkyl) or -N(alkyl)(alkyl).

AA_{c} may be any gene-encoded amino acid. AA_{c} may also be an amino acid residue which is not encoded by the gene, such as hydroxyproline (HP), 4-aminobutyric acid (4ABu), phenylglycine (PG or φG), and the like. AA_{c} is bound to the remainder of the compound of formula 1 through a peptide bond.

For compounds of the invention wherein X is substituted benzyl, whether in the form of the free acid or salt of formula 1 or as esters, amides or cycloamido forms, specificity for various subtypes of human GSTs can be controlled by varying the para substituent on the benzyl moiety. Embodiments of X which contain hydrophobic para substituents such as t-butyl or methyl or benzyl, bind preferentially to human GSTs of the µ (M1) class, such as GST-M1a-1a. Electron withdrawing substituents at the para position, such as nitro or chloro preferably bind π (P1-1) enzymes. Plots of Hammett sigma/meta values against the log fraction of isoenzyme bound show a linear correlation but no clear correlation appears when sigma/para values are used. Some steric bulk appears necessary to bind GSTs generally; benzyl substituents which are unsubstituted or contain only fluoride substitutions do not bind appreciably.

The compounds of the invention may include the alkyl or arylalkyl esters or alkyl or arylalkyl amides as their salts, or may be in the amidocyclic forms. Alkyl esters of the free carboxyls are esters of the straight- and branched-chain alkyl alcohols (1-6C) such as methanol, ethanol, isopropanol, t-butanol, n-hexanol and the like. Suitable alkyl (1-6C) amides are those of primary straight- or branched-chain alkyl amines, such as methylamine, ethylamine, n-propylamine, isopentylamine, and isohexylamine. Arylalkyl esters may contain 7-12C and include, phenyl-lower alkyl derivatives such as benzyl, 2-phenylethyl, 2-phenylpropyl, and the like. The phenyl group may be unsubstituted or may be substituted with 1-2 substituents, such as methyl, chloro and the like which do not materially effect the properties of the invention compounds. The esters and amides are prepared using conventional techniques, with suitable protection of any alcohol or amino functional groups in the substrate compound of formula 1.

It should be noted that the compounds may be prepared as either the mono- or diesters or the mono- or diamides (or triesters or triamides, depending on the choice of AA_{C}). Thus, the invention includes esters wherein only one of the carboxyl groups is esterified, where two carboxyl groups are esterified or where all carboxyl groups (if more than two) are esterified. Similar embodiments are applicable to the corresponding amides. In addition, the compounds may be prepared as mixed esters/amides wherein one carboxyl is an ester and another an amide.

Salts of the compounds of the invention may be formed of inorganic bases such as sodium or potassium hydroxide or of organic bases such as caffeine or piperidine to form the basic salts of the free carboxyl groups or may be formed from organic or inorganic acids to obtain the acid addition salts of free amino groups. Thus, the salts may be of inorganic bases such as sodium hydroxide, calcium hydroxide, ammonium hydroxide, magnesium hydroxide, and the like, or of organic bases such as trimethylamine, pyridine, pyrimidine, lysine, caffeine, and the like. The acid addition salts may be formed from inorganic acids such as hydrochloric acid, hydrobromic acid, sulfuric acid, phosphoric acid, and the like, or from organic acids such as acetic acid, propionic acid, glycolic acid, pyruvic acid, oxalic acid, malic acid, tartaric acid, citric acid, benzoic acid, cinnamic acid, mandelic acid, salicylic acid, and the like.

Salts are formed in standard protocols by treating with the appropriate base or acid at a temperature of from about 0°C to about 100°C, preferably at room temperature either in water alone or in combination with an inert water-miscible organic solvent such as methanol, ethanol or dioxane.

Cyclic forms of the compound of formula 1 are prepared by bridging free amino and free carboxyl groups contained in the substrate compound. Formation of the cyclic compounds is conducted conventionally by treatment with a dehydrating agent such dicyclohexyl carbodiimide by means known in the art *per se*, with suitable protection if needed.

In most embodiments of the compounds of formula 1, the cycloamide is formed between the free amino group present at the amino terminus on Y with the carboxyl group at the carboxy terminus represented by AA_{C}. However, in those limited embodiments wherein AA_{C} contains a side-chain amino group (such as those wherein AA_{C} is lysine) alternative forms of the cycloamido compounds may also be prepared involving the side-chain amine of AA_{C} and the side-chain carboxyl of Y. Suitable protecting groups can be used to direct the cycloamidation as is known in the art.

The compounds of the invention can be further characterized by designating the identities of Y, n, X and AA_{C}. In order to designate Y, the carbonyl group shown adjacent the Y substituent in formula 1 is conveniently included in the designation; if this is the case, the various embodiments of "Y-CO" include γ-Glu, β-Asp, Glu, Asp, γ-Glu-Gly, β-Asp-Gly, Glu-Gly, and Asp-Gly, which in the one-letter amino acid codes can be symbolized as γE, βD, E, D, γE-G, βD-G, E-G, and D-G, respectively.

The various substituents designated by X can be noted by standard abbreviations, and their inclusion in the tripeptide analogs of the compounds of the invention symbolized by C(X) when n=1 or C(X)(X) when n=2. In those compounds of formula 1 wherein n equals 2, the sulfur of the cysteine residue will have a positive charge and exist as a sulfonium ion.

The notation for AA_{C} may also be in the standard one-letter amino acid abbreviation code or other suitable abbreviation for non-gene encoded amino acids.

Thus, using this notation, suitable compounds of formula 1 include:
γE-C(Bz)(Me)-G,
βD-C(Trt)-A, γE-C(p-ClBz)-G,
E-C(Pr)(Me)-V, γE-C(p-NO₂Bz)-PG,
γD-C(Hx)-PG, γE-C(p-MeBz)-V,
E-G-C(Bz)(Et)-4ABu, γE-C(p-BuBz)-βA,
D-G-C(Hx)-F, γE-C(p-MeOBz)-βA,
γE-C(Hx)(Me)-N, γE-C(p-EtSBz)-PG,
γE-C(Trt)-N, γE-C(p-Me₂N-Bz)-βA,
γE-C(o-BuBz)-PG
and the like.

These compounds of formula 1 are cyclized or esterified/amidated to obtain the compounds of the invention.

### Features of the Invention Compounds

The compounds of the invention are useful as solution-phase or immobilized inhibitors of enzymes, such as glutathione-S-transferases (GSTs), which utilize glutathione as substrates. Such inhibition may be desirable both in analytical and therapeutic contexts.

As therapeutic agents, the esterified compounds of the invention are most useful since permeation of the cell membrane is best achieved by these less hydrophilic forms. Particularly preferred esters are the benzyl esters and derivatized benzyl esters. Also preferred are the ethyl esters.

Thus, for use *in vitro* in cell culture, for example in screening assays, the preferred compounds of the invention are the esterified forms. In addition, both the esters and amides offer the opportunity to modify the invention compounds in ways that affect their suitability as therapeutic agents, as diagnostic reagents, and as ligands for coupling to solid supports. The ester or amide groups may contain additional useful ligands such as hydrophobic moieties to enhance cell permeation, reactive groups to facilitate linkage to other substances, and additional ligands such as antibodies or fragments thereof, carriers to enhance immunogenicity and the like. Further, the compounds can be modified by including substituents such as chelating agents which facilitate purification by immobilized metal ion affinity chromatography or substituents which alter the solubility of the peptide or which affect pharmacokinetics such as the addition of polyethylene glycol.

Thus, the esters and amides of the compound of formula 1 offer distinct advantages over the free acid or salt forms in a variety of contexts.

The cycloamido forms of the compound of formula 1 offer the advantage of enhanced specificity and selectivity among target GSTs. The cyclic forms in general are superior as chromatographic ligands to the open chain forms. In addition, their inhibition characteristics are modulated by this feature.

The compounds of the invention that contain substituted benzyl as the embodiment of X are advantageous in that they offer the opportunity systematically to control the selectivity of the compounds for the various GSTs. For instance, as shown in Example 8 herein, the binding specificity among the µ and π groups of human GSTs is controlled by the nature of the para-substituent on a benzyl moiety attached to the sulfur atom of the Cys residue of certain compounds of the invention. There is a correlation in binding specificity with sigma (meta) values of the para-substituents on S-benzyl Cys moieties.

### Use as Chromatographic Ligands and Analytical Reagents

The invention compounds also are useful individually as diagnostic chromatographic supports and chromatographic tools. The use of these compounds in affinity chromatography, for characterization and for purification, as well as in diagnostic assays, is particularly important in the case of human GSTs. While panels of the invention compounds are most convenient in determining the GST complement of tissues, individual compounds can be used to detect the presence of GSTs in general, or of GSTs of a particular type in humans, as well as in purification of particular types of human GSTs. Thus, as shown in Example 8 below, various compounds of the invention have different propensities for binding human GSTs of the µ (M1) and π (P1) classes.

The compounds of the invention, or the compounds of formula 1 alone as the free acids or salts, can be used as chromatographic ligands for purification and characterization of human GSTs collectively, for purification and identification of human classes of GSTs or for purification or separation of individual human GST enzymes, depending on the choice of ligand.

For use as affinity ligands on chromatographic support, the compounds that include formula 1 are coupled to a suitable solid matrix, such as Sepharose, polyacrylamide, silica, and the like using standard coupling techniques. The noncyclic forms of the compounds of the invention contain at least amino and carboxyl functional groups which can be derivatized to suitable linkers or directly to supports. Depending on the nature of the solid support, the coupling of the affinity ligand may employ direct covalent coupling or may require the use of homo- or heterobifunctional linkers such as those available from Pierce Chemical Company (Rockford, IL). It may also be desirable to distance the affinity ligand from the surface of the support in order to render it more accessible. Such distancing can be accomplished using spacer arms, as is generally understood. Further, the support may be treated with an inert material, such as, for example, human serum albumin, so as to minimize unwanted interactions, especially if the support is to be used for the chromatography of biological samples.

The derivatized chromatographic supports may take advantage of the diversity of the compounds which include the structure of formula 1 by coupling the solid support to more than one such ligand. The ligands can be arranged in a gradient, at random as a mixture, or in any predetermined pattern desired. In addition, combinations of the compounds of the invention can be used in chromatographic applications wherein a balance is achieved between one or more compounds which serve as affinity ligands and one or more compounds which are used as competitors to the affinity ligand to effect elution of the materials subjected to chromatographic separation or characterization. Materials of varying affinity for the moieties subjected to the chromatographic technique are used for the ligands and the eluting agents.

The chromatographic supports derivatized to compounds that include formula 1 may then be used for preparative separation of human GSTs or other enzymes for which the ligands have affinity, other enzymes utilizing glutathione as substrate, antibodies which react with glutathione, or other moieties which bind with moderate affinity to the ligands. Chromatographic supports coupled to the compounds of the invention may also be used in diagnosis to determine the presence, absence, quantity or nature of materials in biological or other samples suspected to contain materials having similarity in structure to glutathione.

The suitable compound containing the structure of formula 1 useful in such separations or analyses may be evident from the nature of the analyte or material whose preparation is desired, or may readily be determined by preparing a diverse panel of the compounds and screening the panel for the most effective affinity ligand.

The compounds containing the structure of formula 1 may also be used for detecting the presence or absence of human GSTs in general or for a particular class or isoenzyme thereof. Protocols for such assays mimic those used in immunoassay; these protocols are generally applicable to any assays which involve specific binding partners where the specificity of the assay depends on the specificity of the binding. Thus, in such assays, the compounds including the structure of formula 1 play the role of antibodies or other immunoreactive agents specific for the analyte, in this case the relevant GST. Such protocols include sandwich assays, competition assays, direct binding assays and the like with a wide variety of detection means for formation of "immunocomplexes" (in this case a complex between GST and the compound containing the structure of formula 1). Such detection means include florescent tags, enzyme labels, radioactive labels and combinations of these.

### Panels

The panels of the invention are constructed of at least five diverse tripeptide analogs of glutathione of formula 1, wherein X, AA_{C} and Y are as defined above but wherein X can also be H. The panels are most useful when maximally diverse in character. This diversity can be supplied by varying the natures of Y, AA_{C} and X. In general, by using X substituents, for example, of varying hydrophobicity, a range of such hydrophobicity characteristics can be obtained. X is also a convenient substituent for variation of the Hammett Constants (sigma/meta and sigma/para) diversity of the resulting compounds. The diversity resulting from variation of AA_{C} is somewhat less focused; that provided by variations in Y is limited by the limitations on that structure.

The Hammett Constants refer to the values analogous to those obtained showing the electronic influence of substituents on the ionization constant of benzoic acid. As a result of this early work, numerical values have been assigned to a large array of different substituents. Tables of such values for various substituents are given, for example, by Ritchie, C.D. et al., Prog Phys Org Chem (1964) 2:323.

The construction of a series of the analogs of the invention can be made which systematically varies the value of this parameter by, for example, utilizing as the embodiment of X, a benzyl group which is further substituted with an additional substituent at the para position, such as nitro, chloro, methoxy, or methyl.

The steric characteristics and the resulting properties of the compounds of formula 1 which are members of the panel can also be controlled by cyclization of one or more members of the panel.

For example, a two-dimensional matrix of the glutathione analog tripeptides of the invention forming a suitable two-dimensional panel can be constructed by varying the AA_{C} component from a small hydrophobic residue to a large hydrophobic residue to a positively charged residue to a neutral residue and finally to a negatively charged residue, thus influencing, in the latter three analogs, the inductive electronic effect. Similarly, the X substituents can be varied in the second dimension over the same range from small hydrophobic to large hydrophobic to positively charged to neutral to negative. The resulting matrix provides a suitable panel for use in, for example, determining suitable compounds to use as adsorbents.

Particularly useful embodiments of X are the substituted benzyls wherein it can be shown that variation of the hydrophobicity and electron donating or withdrawing capacities of the para substituent has a systematic correlation with ability to bind certain classes of human GSTs.

### Use of the Invention Panels

The panels are useful in determining the differing complements of the glutathione-S-transferase (GST) isoenzymes as they occur in normal (as compared to unwanted) cells or tissues. By "GST complement" is meant the pattern of levels of GST isoenzymes that is present in such cells or tissues or which is genetically programmed in such a manner that induction or repression of expression levels of such GSTs is manipulable. As explained in the Background section above, GSTs are homo- or heterodimers formed from subunits, of which at least seven are well known. In addition, although these isoenzymes have been classified broadly, individual members within each class may differ from individual to individual due to genetic variation. The properties of the various isoenzymes differ with respect to a series of measurable parameters including substrate specificity, susceptibility to inhibition, binding to specific reagents, and inducibility of expression.

### Use of the Invention Panels to Determine GST Profiles -- Background

Perhaps the most easily understood approach to determining the GST isoenzyme complement of an unknown tissue sample presumes a reference set of all GST isoenzymes which have reactivity profiles that have been or can be determined. Thus, assuming a high enough resolution separation, for example, using any separation technique, analogous, for example, to high resolution chromatographic focusing, an elution pattern is referenced to a series of known enzymes. Other methods for separating and characterizing the known isoenzymes could include the use of antibodies that have been prepared to specific isoenzymes, such as those established for several GST human isotypes and used to assess GST content of these isotypes in candidate tissues (Howie, A.F., et al., Carcinogenesis (1990) 11:451-458; Beckett, G.J. Clinica Chimica Acta (1984) 141:267-273). Gel electrophoresis separations can also be used. The location of the GST bands following electrophoresis under nondenaturing conditions can be determined, for examples, by the method of Ricci, G., et al., Anal Biochem (1984) 143:226-230. The location of various isoenzymes resulting from chromatographic separations can be detected using substrates common to all isoenzymes, such as 1-chloro-2,4-dinitrobenzene (CDNB). Indeed, the distribution of activity as assayed by CDNB in various tissues has been conducted by Pickett, C.B., and Lu, A.Y.H., Ann Rev Biochem (1989) 58:743-764.

The use of these direct separation methods to obtain a pattern of GST isoenzyme distributions in cells and tissues of interest can be used to obtain a GST complement for such cells and tissues that may be useful in the design of therapy, provided that each of these isoenzymes has a reactivity profile which has been determined previously following separation techniques permitting isolation of the individual isoenzyme with retention of activity. Such a reactivity profile would take account of the substances which are effective substrates, substances which are effective inhibitors, and substrates which are effective inducers or activators of GST activity. Once the GST isoenzyme is identified and quantitated by virtue of its position in the elution pattern or electrophoretic gel, for example, reference is made to the reactivity profile of the known and previously isolated isoenzyme in order to predict or design treatment protocols.

This method, while readily comprehensible, is not practical due to the large number of GST isoenzymes that are potential candidates for inclusion in the complement and due to the mutability of the repertoire of GST isoenzymes per se. Thus, a polymorphism in the population of available GST isoenzymes is likely to result in a protein with unaltered mobility, for example, in the elution pattern, but with altered substrate specificity or inhibition pattern, or vice versa. In either case, the results of the matching of the position in the elution pattern to the set of reference characteristics would give misleading results.

A somewhat improved result can be obtained by utilizing multiple separation techniques, it being less likely that mobility would be unaffected in multiple separation systems as compared to one. Such a system is generically illustrated by a simple hypothetical system in which a first sorbent, P1, produces four isoenzyme peaks in its elution pattern and five are obtained on a second sorbent, P2. If the substrate specificity patterns (for substrates A, B and C, for instance) indicate that a given peak from P1 (identified, e.g., as 1.2) is substantially the same in substrate profile as a particular peak separated on sorbent P2 (peak 2.4), then one would assume that a tissue sample which provided a peak at 1.2 in sorbent P1 and at 2.4 in sorbent P2 would be highly likely to have a reactivity profile (with A, B and C) the same as that of the isoenzyme forming peaks 1.2 and 2.4.

The technique illustrated in this hypothetical system can be applied using, as affinity-based sorbents, the novel glutathione tripeptide analogs of the invention or one such novel tripeptide in combination with an additional analog or panels of analogous glutathione analogs with diverse properties. It is believed that to perform effective characterization, a panel of at least two, and preferably three, and more preferably 5 such analogs should be used. The members of the panel should have properties which are sufficiently diverse to assure discrimination among the various GST isoenzymes in the complement.

If the separation technique preserves enzymatic activity, the reactivities of each enzyme against potential drugs can be directly determined. Nondenaturing separations in the art, however, suffer from either a lack of resolution or from hypersensitivity to structural changes, making peak identification too problematic for effective guidance of therapy. Ion exchange chromatography, for example, can be used as a step to purify individual GST isoenzymes, but has inadequate or inappropriate resolution as an analytical tool. IEF, another technique available in the art, is prone to generation of numerous extraneous peaks due to *in vivo* or *in vitro* posttranslational modification of the protein, and there is no necessary linkage between such structural changes and functional variation.

Thus, determination of the activity profile of the GST complement in cells or tissues by separating the individual isoenzymes using prior art methods and determining an activity profile for each of them against all possible chemotherapeutic drugs would be laborious, but is enhanced by the availability of the novel GSH-analog tripeptides of the invention. The compounds of the invention permit separation without denaturation of the GST enzymes.

A more efficient approach takes advantage of profiles of GST isoenzyme complements which simultaneously measure specific binding activity and reactivity characteristics. These profiles, designated survey of characteristics profiles, or "SC" profiles, permit the determination of a reference set of SC profiles which include information on substrate specificity, induction in response to specific inducers, and the like, as well as additional binding or electrophoretic mobility characteristics. By applying computational methods to comparison of these profiles, the requisite information for the design of therapeutic modulators and accompanying protocols and for prediction of success or failure of proposed protocols can be obtained for significantly larger numbers of specimens than by prior art methods, as is needed to provide an adequate guide for therapy. Among the parameters that can be used for obtention of an SC profile is the ability to bind to members of the panels of tripeptide GSH analogs of the invention, or the effect of such panel members on activity.

In this approach to determining the GST complement of unknown samples of cells and tissues, advantage is taken of pattern recognition techniques. In connection with this approach, what is here termed an SC profile is determined generally with respect to a panel of reagents which react specifically and differentially with the various GST isoenzymes. This is analogous to the determination of profiles obtained by cross-reaction immunoassay, and refers to any pattern of reactivity of a candidate GST isoenzyme or mixture of isoenzymes with a panel of reagents. Thus, the SC profile may be determined with respect to turnover rates for various substrates; effective levels of inhibitor concentration for various inhibitors; levels of inducers required to induce the expression of the gene for the GST isoenzyme in the context of a particular host cell; mobility in electrophoresis in the presence or absence of inhibitors or substrates; elution times from paralog or other affinity columns or, indeed, the classical pattern of binding with a panel of antibodies. The SC profiles obtained for individual GST isoenzymes or mixtures of isoenzymes at various concentration levels can be manipulated in various ways, described herein, to provide a reference set against which SC profiles of unknown samples can be compared.

In general, the SC profile will provide values for each of a panel of "information channels" wherein each information channel describes a characteristic of the GST complement or standard, such as the binding affinity for an antibody, a substrate affinity, an elution time or the like. At least some of the information channels should relate to values that vary with concentration of the GST.

Determination of the GST complement for cells or tissues is useful *per se* in diagnosis and characterization of samples. In addition, for use in the design of embodiments of the strategies to impair or destroy unwanted tissue, the SC profiles must provide information related to GST activity so that activity differences between normal and unwanted tissues can be determined. Thus, the SC profiles of the unwanted tissue must be readily comparable to the reference standards which, in turn, must at least in part be based on reactivity patterns that will aid in the design of therapeutic modulators and the selection of drugs or prodrugs. For this application, at least a portion of the reference profiles must be grounded in substrate turnover rate data, inhibition data, or data relating to induction of isoenzyme production level, or any other reactivity which will permit manipulation of the GST isoenzyme in situ. The panels of the invention may be used to determine such effects on activity. The combination of chromatographic separation which permits activity to be retained using the novel compounds of the invention along with preparation of SC profiles with regard to reactivity-affecting reagents for these standards is one approach to obtaining the needed data.

The SC profiles of the reference standards and of the unknown samples are determined with respect to panels of "specifically reactive reagents." These reagents may include a variety of substances, including paralogs, substrates, inhibitors, inducers, antibodies, as well as "reagents" which are actually techniques such as gel electrophoresis or affinity chromatography where the extent of reactivity is determined as electrophoretic mobility or elution time. Thus, "specifically reactive reagents" is not limited to those reagents which effect a chemical reaction, but includes any reagent or technique that permits a characteristic parameter to be obtained for the sample with respect to that reagent.

Of course, the panels of the analogs of the invention can be used as "specifically reactive reagents" either as binding agents, chromatographic supports, or as inhibitors of enzyme action in solution. The comparative ability of these compounds as members of the panel to inhibit the enzymatic reactions catalyzed by GST can be used as a characteristic SC profile, as can elution patterns from columns containing the tripeptide analogs as affinity ligands.

### Determination of Reference SC Profiles

While reference standards for some purposes, as described below, can be prepared directly from normal tissue of a particular subject, it is also useful to provide a databank of SC profiles for a variety of previously isolated GST isoenzymes with characteristic reactivity patterns. By matching these reactivity patterns with those from biopsy samples of the unwanted tissue, the appropriate design for therapeutic modulators and choice of prodrugs or toxins can be made.

U.S. Patents 4,963,263 and 5,133,866, the disclosures of which are incorporated herein by reference, describe panels of paralog affinity reagents that are useful in chromatographic separations of closely related substances. The panels of the invention are similarly diverse. Paralog-type panels using the GSH tripeptide analogs of the invention can be conveniently used in the preparation of affinity supports for the separation of various GST isoenzymes in purified form while, in each case, retaining the activity of the native isoenzyme. Unlike the reverse-phase HPLC or Western blot methods of the prior art, the separated isoenzymes prepared using chromatography based on affinity for the compounds of the invention behave in a manner virtually precisely similar to that of the isoenzymes as they occur in nature. For each such purified isoenzyme, then, a SC profile with respect to reactivity of substrate or other activity-affecting reagent such as those represented by the compounds of the invention can be constructed. A helpful data bank of a large number of SC profiles characteristic of these purified isoenzymes can then be retained and stored in mathematically or computationally accessible form for comparison to samples obtained from the unwanted tissue.

Panels of tripeptide glutathione analogs, at least one member of which contains the structure of formula 1, can be used as the basis for the collection of information channels which provides the SC profiles for the reference set. Conjugation of known isoenzyme specific substrates to the members of the panel or conjugation of the members of the panel directly to sorbent further increases the systematic diversification of binding properties. Profiles for standards and unknown samples can be obtained and compared using the panels of the invention in appropriate configurations, such as affinity columns. Thus, particularly useful are panels of diverse tripeptide analogs including at least one analog containing formula 1 wherein X is a mono- or disubstituted or unsubstituted hydrocarbyl (1-20C) moiety optionally containing 1 or 2 nonadjacent heteroatoms (O, S or N), and wherein said substitution is selected from the group consisting of halo, NO, NO₂, NR₂, OR, and SR, wherein R is H or lower alkyl (1-4C) and

AA_{C} is an amino acid coupled through a peptide bond to the remainder of the compound of formula 1.

### Determination of GST Complement

In general, two different approaches can be made to determine the GST complement of an "unknown" sample cell or tissue. First, as described above, using general techniques presently practiced in the art, the individual isoenzymes contained in the sample can be separated using affinity supports and tested individually for their patterns of activities. The individual isoenzymes from the sample can be obtained and then independently assessed for their substrate specificity, inhibitor specificity and for identifying substances which induce the activity or production of the isoenzyme.

In a second, less laborious, approach, pattern recognition techniques are employed to obtain an instant readout for samples of either or both unwanted and normal tissue for an individual subject by matching these patterns against a reference set prepared as above. In this approach, less volume of sample is required and no separation is necessary. This method is also useful when applied to tissue slices using histochemical staining for GST activity.

With respect to the first approach, a modification of the separation method used by Vos, R.M.E., et al., Biochem Pharmacol (1988) 37:1077-1082, can be used. In this method, the cytosol fraction from a complete rat liver was subjected to an affinity column of S-hexyl GSH Sepharose used as an affinity reagent for GSTs as a group. The eluted GST mixture was concentrated and separated by chromatofocusing on a mono-PHR 5/20 column (Pharmacia FPLC system). The individual isoenzymes were collected in separate fractions and analyzed. Fractions were identified by their position in the elution profile, their subunit molecular weight, and specific activities toward 1-chloro-2,4-dinitrobenzene, which is a substrate for most known GSTs.

By using as the affinity ligand a paralog chosen from among the compounds of the invention, milder conditions can be employed, and more active forms of the GST isoenzymes can be recovered. These are then tested for substrate specificity, etc.

As described above, one might consider the possibility of simply using arbitrary separation technology such as that of Vos that provides an elution pattern characteristic of the various GST isoenzymes, and matching the elution pattern for cells or tissue of unknown GST complement with the preset elution pattern to determine the nature of the GST complement in the unknown. One problem with this approach, however, lies in the genetic mutability of GST, so that it is difficult to make reliable matches that will retain the inferred characteristics and thus be assured to have similar reactivity patterns. The genetic mutability of the isoenzymes as well as their sensitivity to posttranslational modifications is very likely, in any particular case, to have a profound effect on the substrate specificity, inhibition patterns, and the like, as well as in binding and physical characteristics, such as pI. There is no guarantee that a correlation will exist between reactivity variation and physical property or binding variation; indeed, the probability is that the effects will not be correlated. As described above, this problem can be mitigated by using multiple affinity reagents, also provided by the invention compounds.

In the pattern-matching approach a more reliable assay is conducted by comparing the profile of reactivity of an unknown sample with a set of reference SC profiles. The application of this approach to the determination of the composition of analyte in general is described in copending application Serial No. 07/678,049, filed 2 April 1991, the disclosure of which is incorporated herein by reference. According to the techniques described in this application, a predetermined plot of profiles obtained from samples of known analyte composition is used as a reference with which an SC profile of the sample to be tested can be compared. Generally, a panel of 2-10, preferably 4-6, different specifically reactive reagents is first used to provide profiles for samples of known compositions. In the referenced application, specific binding assays were used where there was cross-reactivity by the candidate analytes across a panel of binding agents, and the profiles were obtained by measurement of inhibition values for binding of a known binding partner by various analytes. The collection of profiles is then treated mathematically by any of a number of techniques to generate a readable comparison with the corresponding SC profile of an unknown sample.

For use in determining the GST complements needed to practice the therapeutic methods of the invention, the analogous SC profiles can be determined using either isolated GST isoenzymes or mixtures thereof which contain known compositions or both. The specifically reactive reagents must include, as a panel, at least one, preferably three, and more preferably five GSH analogs of formula 1, along with additional reagents, if desired. Such additional reagents may include a series of known substrates wherein turnover rates are measured. Suitable substrate candidates include, for example, ethacrynic acid, bromosulfophthalein, cumene hydroperoxide, BCNU, chlorambucil, trans-stilbene oxide and the like.

Also available for use as specifically reacting reagents which are members of the panel to provide a SC profile are inhibitors which interact with the GST isoenzyme at various levels. These inhibitors include, for example, piriprost, Cibacron Blue, and hematin. Antibodies which are specifically immunoreactive with the various isoenzymes can be used, as well as paralog-type affinity reagents. If the profile is to provide a basis for therapeutic strategy design, it is preferred that at least some members of the panel must be descriptive of the enzymatic activity of the GST.

An additional technique for obtaining SC profiles is analogous to that described by Takeo, K., et al., J Immunol (1978) 121:2305-2310. In this approach, differential electrophoresis in the presence of various binding agents for the individual proteins permits measurement of a mobility value. In the specific application described by Takeo, measurements of dextran-specific myeloma proteins in polyacrylamide gel electrophoresis were made, showing retardation when the dextrans were added to the separating gel, which retardation could be reversed by adding the hapten isomaltose oligosaccharide. In using this approach, a series of mobilities depending on the choice of retarding agent, for example, could be obtained for known compositions. This technique may be applied by using the novel compounds or panels of the invention as the retarding or mobilizing agents.

In one preferred method for determining the GST complement of biopsies, a series of HPLC columns is constructed using the known GST substrates studied by Mannervik, B., et al., Proc Natl Acad Sci (1985) 82:7202-7206. These substrates are conjugated directly to the column supports or are attached to the GSH analog variants described by Adang, A.E.P., et al., Biochem J (1990) 269:47-54. A series of 50-100 different columns resulting from the various possible combinations of substrates with GSH analogs of formula 1 represents a series of candidate sorbents. These sorbents are tested to select those of maximal diversity in properties by utilizing each for the separation of a mixture of known GST isoenzymes. The four or five columns with the greatest differentiation capacities are then chosen as panel members for determining SC profiles in unknown samples and in standards.

Thus, rather than displaying the separations as elution patterns on each individual sorbent, the data are rearranged so that the capacity for adsorption to each sorbent represents an information channel in the SC profile of the isoenzyme. The reactivity pattern with respect to inducers, activators, substrates, and inhibitors are also determined for each isoenzyme and used as an information channel. The completed profile for each known isoenzyme is then used as a member of a reference set. Additional members of the reference sets are determined by utilizing samples from normal tissues and evaluating the values assigned to the same set of information channels. The corresponding profiles of biopsy samples from unknown, unwanted tissues are then compared against this reference set.

The profiles for known compositions are stored in computationally accessible form for comparison to profiles similarly determined for unknown samples. Thus, kits can be provided for determination of the GST complement of unknown samples which include the test panel members used in determination of the reference profiles along with instructions for SC profile determination of the unknowns. Suitable software to access the reference profiles may also be included. The GST complement can be used to characterize the sample tested and, if appropriate, may be used to design therapy.

For diseased tissue, the appropriate strategy can be selected for treatment. The complement may be evaluated to determine whether or not standard treatment protocols will be successful when applied to the unwanted cells or tissues or may be used for the design of different protocols including the choice of toxin or prodrug and the inclusion or noninclusion of a therapeutic modulator.

### Synthesis of the Novel Tripeptide Analogs

The novel tripeptide analogs of the invention or additional tripeptide analog members of diverse panels can be synthesized using means generally known in the art, but using modifications which render these general methods applicable to the desired tripeptide. Although solid-phase synthesis methodologies can be used, liquid-phase peptide synthesis appears superior for these short peptides. The Fmoc reagents originally developed for solid-phase synthesis can be applied to a solution-phase method for producing 100 mg quantities of the tripeptide analogs.

The intermediate protected dipeptides and tripeptides synthesized using solution-phase Fmoc chemistry are isolated by chromatography on silica gel, and deprotected in mild base, thus allowing synthesis of acid-labile thioconjugates (Iselin, B., et al., Helv Chem Acta (1955) 38:1508-1516). The analogs can be purified and recovered, or the crude product mixtures may be directly coupled to solid support to provide affinity-derivatized supports (Sundburg, L., et al., J Chromatog (1974) 90:87-98).

In those circumstances where ester of the C-terminal amino acid AA_{C} is not available, the ester is made by synthesizing the N-Fmoc-protected amino acid (Atherton, E., et al., in "Solid Phase Peptide Synthesis," IRL Press, Oxford, England (1989), pages 47-61) and then esterified by treatment with the desired alcohol in the presence of concentrated sulfuric acid (Benoiton, L., Can J Chem (1962) 40:570-572). Nonesterified materials are removed by extractions with mild base and the desired N-Fmoc amino acid ester is isolated by evaporation.

The sulfur-functionalized Fmoc cysteine derivatives are made in a one-pot procedure by treating cysteine with Fmoc-OSu as pH 9 and then treating this mixture with the appropriate alkylating agent.

The synthesis is conducted as shown in Reaction Scheme 1.

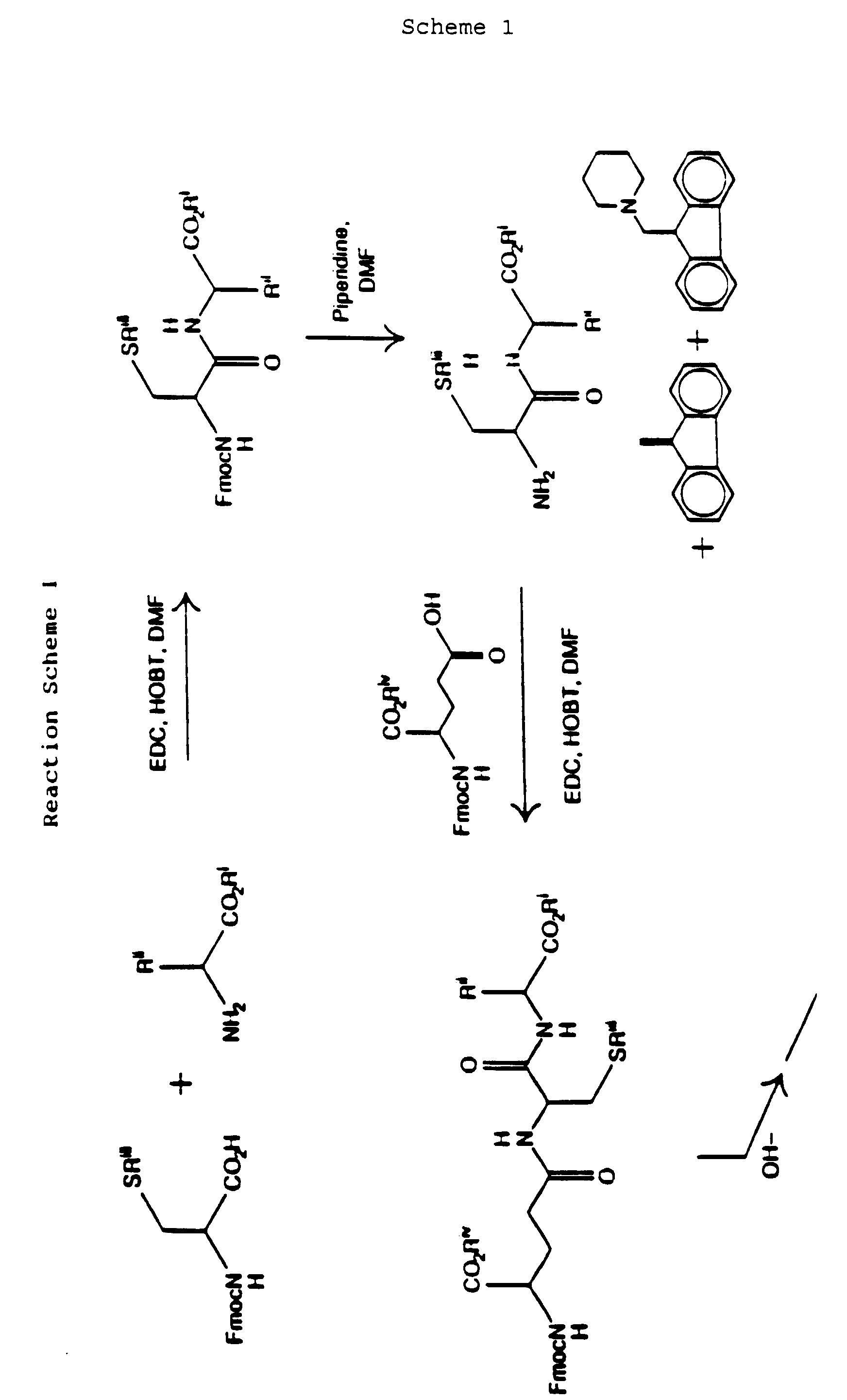

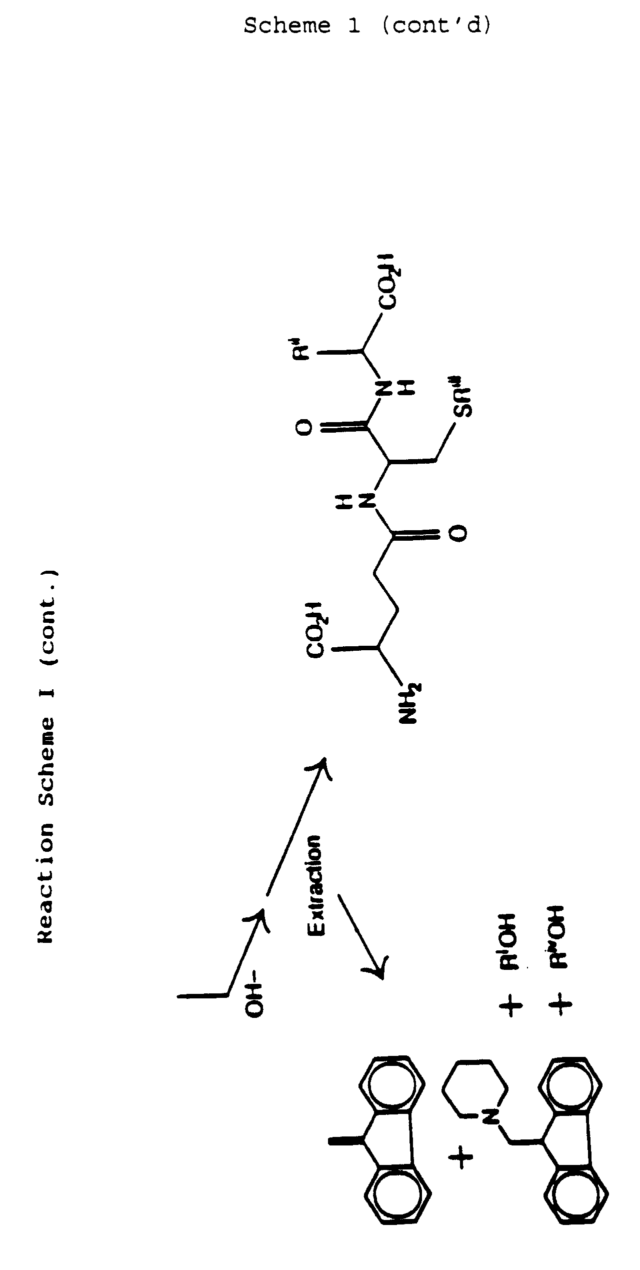

Coupling the cysteine derivative to the C-terminal amino acid is accomplished with the water-soluble carbodiimide EDC (Sheehan, J., et al., J Org Chem (1961) 26:2525-2528) and HOBT (Konig, W., et al., Chem Ber (1970) 103:788-798) (added to retard undesired racemization and speed up the reaction) in DMF. After coupling is complete, usually about 1 hr at r.t., the mixture is reduced *in vacuo* and poured into KHCO₃ solution (John Hughes, private communication). This step extracts most of the DMF and EDC and EDC urea, as well as some of the Fmoc-cysteine derivative which did not couple. The resulting gummy residue is retained by decanting off the liquid. This crude dipeptide is dissolved in EtOAc and washed with 1N hydrochloric acid and water to remove any remaining uncoupled C-terminal amino acid ester, as well as residual EDC and EDC urea. The solution is concentrated and the dipeptide purified by chromatography.

The recovered dipeptide is then treated with 25% piperidine in DMF for 30 min to remove the Fmoc group. The dibenzofulvene or its piperidine adduct resulting from Fmoc removal should not affect the results of the next coupling (Atherton, E., et al., J Chem Soc Perkin Trans (1981) 1:538-546). Any excess piperidine, however, must be removed, which is accomplished by repeated co-evaporation with DMF until the odor of piperidine is no longer detectable in the deblocked material. The second coupling is then performed with the glutamic acid derivative followed by the same workup as for the dipeptide.

Fmoc glutamic acid α-benzyl ester is made in good yield and purity from commercially available glutamic acid α-benzyl ester. Fmoc-glutamic acid α-tert butyl ester, also commercially available, can also be used, but this requires a separate acid treatment step in the workup. There are solubility problems with some of the protected tripeptides during this step, and impure, partially deprotected products are often obtained.

The material produced by the coupling of the glutamic acid derivative to the dipeptide and workup contains several chromatographically mobile components. The material that elutes first from the final column is fluorescent, suggesting dibenzofulvene. The putative desired product elutes next along with another UV-absorbing material, which is probably the piperidine adduct of dibenzofulvene. Since similar products are generated and separated from the deblocked tripeptide during the final workup, these contaminants are not removed at this stage.

Once the protected tripeptide (and impurities) are isolated, it is dried under vacuum and treated with 0.25 N NaOH in 3:1 ethanol:water for 18 hrs. This removes the Fmoc and ester-protecting groups. When t-butyl-protected glutamic acid is used, 3N HCl in ethanol/water 3/1 v/v for 3 hr is used to remove the t-butyl group before the base treatment. The acid is removed by rotary evaporation and co-evaporation with ethanol and water, and the same base treatment as above removes the remaining protecting groups. After the overnight base treatment, addition of water and extraction with hexane removes the organic by-products of the deprotection. The aqueous solution of the peptide is acidified and reduced to a solid. Dissolution of the peptide in ethanol and filtration removes the salt. This is evaporated to a foam and subjected to high vacuum overnight.

The compounds are analyzed by HPLC, TLC and FAB mass spectroscopy. While the TLC analysis show good results in most cases, the HPLC results are mixed, partially because the analysis conditions used were not optimized for some of the more hydrophobic (S-alkyl C-terminal valine, β-alanine and 4ABu) peptides.

Racemization during the final deprotection with base may occur in some small amount, particularly with the phenylglycine analog (Bodansky, M., et al., in "Practical Methods in Peptide Synthesis," Springer Verlag, Berlin (1984)). This is less than that which occurs with the sodium-ammonia conditions used previously by Adang, A. et al. (Biochem J (1989) 264:721-724).

Using the techniques set forth above, the analogs of Table 1 were prepared.

**Table 1**

| Compound^{a} | Yield, %^{b} | TLC R_{f}^{c} | M/e^{d} | Loading^{e} |
|---|---|---|---|---|
| γE-C(Bz)-G | 32 | 0.49 | 388.2, 402.2^{f} | 1.0 |
| γE-C(Pr)-A | 23 | 0.71 | 365.2 | 9.0 |
| γE-C(Hx)-A | 17 | 0.76 | 406.2, 428.2^{f} | 4.8 |
| γE-C(Bz)-A | 44 | 0.35 | 412.2, 434.2^{f} | 6.6 |
| γE-C(Trt)-A | 15 | 0.83 | 586.4^{f} | 1.2 |
| γE-C(Me)-βA | 27 | 0.58 | 357.1^{f} | |
| γE-C(Pr)-βA | 27 | 0.41 | 364.1, 386.1^{f} | |
| γE-C(Hx)-βA | 13 | 0.49 | 406.3, 428.3^{f} | 6.0 |
| γE-C(Bz)-βA | 17 | 0.66 | 434.2^{f} | 8.1 |
| γE-C(Trt)-βA | 42 | 0.92 | 564.3, 586.5^{f} | N/A |
| γE-C(Pr)-4ABu | 13 | 0.51 | 378, 400^{f} | |
| γE-C(Hx)-4ABu | 17 | 0.52 | 402.3, 424.3^{g} | 4.6 |
| γE-C(Bz)-4ABu | 23 | 0.70 | 426, 448.2^{f} | 4.0 |
| γE-C (Pr)-V | 23 | 0.67 | 391 | 13.7 |
| γE-C(Hx)-V | 15 | 0.64 | 434.2, 456.2^{f} | 19.5 |
| γE-C(Bz)-V | 26 | 0.73 | 440.1, 462.1^{f} | 6.5 |
| γE-C(Pr)-D | 33 | 0.55 | 408 | 7.0 |
| γE-C(Hx)-D | 25 | 0.68 | 451.2 | 5.9 |
| γE-C(Bz)-D | 22 | 0.59 | 456.1, 478.1^{f} | 2.4 |
| γE-C(Pr)-PG | 14 | 0.64 | 426.4, 448^{f} | |
| γE-C(Hx)-PG | 13 | 0.63 | 468.3 | 4.8 |
| γE-C(Bz)-PG | 11 | 0.61 | 474.1, 496.1^{f} | 2.0 |
| γE-C(Pr)-H | 6 | 0.57 | 429.2 | |
| γE-C(Hx)-H | 30 | 0.61 | 473.3 | 6.0 |
| γE-C(Bz)-H | 11 | 0.58 | 499.3^{f} | 1.0 |

| | | | | |
|---|---|---|---|---|
| ^{a} Standard one-letter AA code, with Me = methyl, Pr = n-propyl, Hx = n-hexyl, Bz = benzyl, Trt = trityl (triphenyl methyl). | | | | |
| ^{b} Moles of deprotected product divided by the moles of Fmoc-cysteine derivative used. | | | | |
| ^{c} TLC R_{f} values for silica plates eluted with EtOAc/pyridine/HOAc/water 5/5/3/1 and visualized with ninhydrin spray. | | | | |
| ^{d} Observed molecular mass, in AMU, usually the molecular weight plus 1. Thioglycerol or nitrobenzyl alcohol matrix. | | | | |
| ^{e} Micromoles of peptide per mL of swollen resin volume (water) | | | | |
| ^{f} Sodium adduct, M + 22. | | | | |
| ^{g} Molecular ion and sodium adduct minus water; MH⁺ - 18. | | | | |

In addition, other compounds of formula 1, including the cycloamido forms of these compounds, as well as the ethyl and benzyl esters thereof, are prepared.

The following examples are intended to illustrate, but not to limit, the invention.

### EXAMPLE 1

### Synthesis of 9-Fluorenylmethoxycarbonyl-4-aminobutyric acid ethyl ester

Forty-five g (0.1339 M, 0.94 eq) of Fmoc-Osu was added slowly to a solution of 14.75 g (0.143 M, 1 eq) of 4-aminobutyric acid (4-ABu) and 20 g of Na₂CO₃ in 300 mL of deionized water and 200 mL of tetrahydrofuran (THF). The pH was monitored and more Na₂CO₃ was added to keep the pH above 8. The reaction was stirred for 2 hr and then acidified with conc. HCl. The resulting cloudy suspension was extracted with 600 mL of ethyl acetate (EtOAc), after which the organic layer was further extracted with 300 mL 0.5 N NaOH. The aqueous layer was rapidly poured into 20 mL of conc. HCl in 500 mL of ice water. The resulting white suspension was extracted with 300 mL of EtOAc, dried over 50 g of Na₂SO₄ and evaporated to 35 g (76% yield) of Fmoc-4-ABu as a white powder. This was dissolved in 500 mL of absolute ethanol and 40 mL of conc. H₂SO₄ was added. After 4 hrs, the solution had become a semi-solid white mass. This was poured into 2 L of water and filtered. The white material was dissolved in 500 mL of EtOAc and extracted once with 200 mL of 0.5 N NaOH, dried and evaporated to give 30 g (79% yield) of total compound, R_{f} 0.71 (20% MeOH in CH₂Cl₂), mp 83-86°.
Anal. Calcd. for C₂₁H₂₃NO₄: C, 71.37; H, 6.56; N, 3.96. Found: C, 71.42; H, 6.67; N, 3.72.

### EXAMPLE 2

### Synthesis of 9-flourenylmethoxycarbonylphenylglycine ethyl ester

In a similar synthetic procedure, 20 g of phenylglycine gave 33.7 g (68% yield) of Fmoc-phenylglycine. 19 g of this product was converted into 10 g (57% yield) of product, R_{f} 0.95 (same TLC system), mp 130-133°.
Anal. calcd. for C₂₅H₂₃NO₄: C, 74.80. H, 5.77. N, 3.49. Found: C, 74.72, H, 5.91, N, 3.20.

### EXAMPLE 3

### Synthesis of 9-flourenylmethoxycarbonylaspartic acid dimethyl ester

Forty-five g (0.134 M, 0.96 eq) of Fmoc-Osu was added to 20 g (0.15 M, 1 eq) of aspartic acid and 20 g of Na₂CO₃ dissolved in 400 mL of water and 200 mL of dioxane. The mixture was stirred for 2 hrs while the pH was maintained at about 9 by the addition of small amounts of Na₂CO₃. Then the cloudy white mixture was poured into 500 mL of ice water containing 40 mL of conc HCl. The white solid was extracted with 500 mL EtOAc and this was mixed with 500 mL of hexane. The mixture was chilled overnight and stirred the next day to give 38 g of Fmoc-aspartic acid as crystals (71% yield) upon filtration and air drying. 10 g (0.28 M) of this product was dissolved in 200 mL of methanol and 20 mL of conc H₂SO₄ was added. The solution was allowed to stand overnight. The mixture was poured into 1 L of water and filtered. The resulting white solid was dried and redissolved in EtOAc. Slow addition of hexane and chilling gave 9 g (83% yield) of product as white needles, mp 78-80°. [a]_{d} = -13.9°.
Anal. calcd. for C₂₁H₂₁NO₄: C, 65.78. H, 5.52. N, 3.65. Found: C, 66.18. H, 5.68. N, 3.69.

### EXAMPLE 4

### Synthesis of 9-flourenylmethoxycarbonyl-S-hexyl cysteine

A. Twenty g (0.127 M, 1 eq) of cysteine hydrochloride and 20 g Na₂CO₃ was dissolved in 800 ml of water under a stream of argon. Two hundred mL of CH₃CN was added, and then 42 g (0.122 M, 0.96 eq) of Fmoc-Osu was added in small portions while the pH was maintained at about 9 by adding 5 g portions of Na₂CO₃. The reaction was stirred for an additional 2 hrs, and 18.6 mL (26.8 g, 0.126 M, 0.99 eq) of 1-iodohexane was added as a solution in 200 mL of CH₃CN. The reaction was stirred for an additional 2 hrs and poured into 1 L of ice water and 50 mL of conc HCl. The white mixture was extracted with 600 mL of EtOAc, and the organic layer was extracted with 2 500 mL portions of 1 N KOH. Each of these was immediately dropped into separate portions of 500 ml of water and 30 mL of conc HCL, and the cloudy mixtures obtained were each extracted with 500 ml of EtOAc. These were each dried over Na₂SO₄ and evaporated. The total yield was 24.6 g (45%). The second fraction (3.5 g) crystallized on standing, mp 101-103°. R_{f} = 0.57. [a]_{d} = -14.3°.
   Anal. Calcd. for C₂₁H₂₃NO₄S: C, 65.42. H, 6.01. N, 3.63. Found: C, 65.53. H, 5.74. N, 2.91.
B. Additional S-functionalized Fmoc cysteine derivatives were prepared as set forth in paragraph A.

### EXAMPLE 5

### Synthesis of Fmoc-glutamic acid α-benzyl ester

Twenty-five g (0.105 M) glutamic acid α-benzyl ester and 25 g Na₂CO₄ was dissolved in 400 mL of water and 200 mL THF was added. 34 g (0.101 M, 0.96 eq) Fmoc-OSu was added in small portions with stirring, and the pH was kept at about 9 by adding more Na₂CO₃ as needed. After 1 hr, the reaction was poured into 500 mL of water and acidified with conc HCl. The white suspension was extracted with EtOAc, dried over Na₂SO₄ and evaporated to a solid mass. This was dissolved in 500 mL hot EtOAc and 300 mL hexane was added. Overnight chilling, collection and air-drying gave 38.7 g (83% yield) of white crystals, mp 110-112°. [a]_{d} = -13.8°. M/e (Rel. inten.): 460.2 (19), 363.4 (8), 345.4 (19), 307.2 (10), 289.2 (12), 238.2 (12), 191.2 (10), 178.2 (89), 165.1 (23), 154.1 (57), 136.1 (48). ¹H NMR (400 mHz), PPM: 1.9 (m, 1H), 2.2 (m, 1H), 2.4 (M, 2H), 4.1 (t, 1H), 4.4 (d, 2H), 4.43 (m, 1H), 5.1 (s, 2H), 5.6 (d, 1H), 7.3 (m, 9H), 7.5 (d, 2H), 7.7 (d, 2H), 9.4-9.6 (broad s, 1H). ¹³C (100 mHz), PPM: 27.5, 30.0, 47.3, 53.5, 67.3, 67.7, 120.2, 125.0, 127.3, 128.5, 128.8, 129.2, 135.2, 141.5, 143.6, 143.9, 156.3, 171.4, 177.8.
Anal. Calcd. for C₂₇H₂₅NO₆: C, 70.57. H, 5.48. N, 3.05. Found: C, 69.71. H, 5.58. N, 2.88.

### EXAMPLE 6

### Synthesis of γ-glutamyl S-benzyl cysteinyl β-alanine

1.5 g (9.76 mmol, 1 eq) of β-alanine ethyl ester hydrochloride was added to 50 mL of DMF and 1.8 mL of DIPEA was added. 3.5 g (8.1 mM, 0.83 eq) of Fmoc-S-benzyl cysteine was added and dissolved by swirling the solution. Next 2 g of EDAC and 250 mg of HOBT were added, and the solids were dissolved by swirling. The mixture was allowed to stand for 1 hr, and was concentrated *in vacuo* to a mobile oil of about 5 mL in volume. To this was added 100 mL of 10% weight/volume KHCO₃ in water, and the mixture was shaken and the liquid removed by filtration. The residue was dissolved in 100 mL of EtOAc, washed with 50 mL of 1 N HCl, 50 mL of water and dried over Na₂SO₄. The solution was filtered and concentrated *in vacuo* to give a foam which was chromatographed using a 2 x 6 cm bed of silica gel packed in CH₂Cl₂. The column was eluted until the first UV absorbing material appeared, then a gradient was run in 1% methanol increments of 100 mL volume to 3% methanol. A strong UV absorbing band eluted after two portions of 3% methanol; these were checked for purity by TLC and pooled and evaporated *in vacuo* to give 4.6 g (83% yield) of Fmoc-Cys(S-benzyl)-β-alanine ethyl ester. Half of this (4 mmol) was dissolved in a mixture of 30 mL DMF and 10 mL piperidine, and allowed to stand for 30 min. The solution was reduced to a solid *in vacuo*, and the process was repeated twice again with 50 mL of DMF. The resulting white solid was subjected to a high vacuum for 1 hr, then it was dissolved in 50 mL of DMF. For the second coupling step, 1.6 g (3.5 mmol, 0.9 eq) of Fmoc-glutamic acid (α) benzyl ester (v) was added, followed by 0.8 g (4.2 mmol, 1.05 eq) of EDAC and 200 mg (1.4 mmol) of HOBT. The mixture was allowed to stand 1 hr, and concentrated *in vacuo* to about 5 mL in volume. This was poured into 100 mL of 10% aqueous KHCO₃ solution and shaken. The liquid was poured off, and the residue was dissolved in 100 mL of ethyl acetate. The organic layer was washed with 50 mL of 1 N HCl, 50 mL of water, and dried over Na₂SO₄. This was reduced to a tar and chromatographed in the same manner as the protected dipeptide. 1.2 g (42% yield) of the protected tripeptide was obtained. This material was dissolved in 30 mL of absolute ethanol and 10 mL of 1 N NaOH solution was added. The mixture was allowed to stand for 18 hrs, and was poured into a separatory funnel with 40 mL of water and 40 mL of hexane. The layers were shaken and separated, and the aqueous phase was washed with an additional 40 mL of hexane. The pH of the water layer was adjusted to about 3 by adding a few drops of conc HCl, and the cloudy solution was reduced to a solid *in vacuo* and subjected to a high vacuum for several hrs. The residue was washed with 2 20 mL portions of absolute ethanol. The ethanol-NaCl slurry was filtered, and the clear solution was evaporated to a tar. The weight was 620 mg (89% yield from the protected tripeptide, 19% from Fmoc-cys(Benzyl)-OH). A TLC plate was run in ethyl acetate/pyridine/water/acetic acid 5/5/3/1 v/v and visualized with ninhydrin spray and heat (Stewart, J. et al., "Solid Phase Peptide Synthesis" (1984) pp. 53-124, Pierce Chemical Co., Rockford, IL, R_{f} 0.66. HPLC analysis showed 74% purity by area integration of UV absorbing material. Fast atom bombardment Mass spectroscopy (FABMS) showed an ion peak at 434.2 M/e, consistent with the tripeptide monosodium salt. Other higher mass peaks were also present, attributable in part to incompletely deprotected peptide.

In cases where the Fmoc protected C-terminal amino acid ester was used instead of the commercially available free amino C-terminal amino acid ester, this material was deblocked with the same procedures as those used to deblock the protected dipeptide above, then proceeding with the first coupling reaction as above.

### EXAMPLE 7

### Derivatization to Sepharose Resin

0.66 g epoxy Sepharose™ 6B (Pharmacia) was swollen with 10 mL water for 15 min, then rinsed twice with 10 mL water in a 15 mL sintered glass funnel. A solution of 100-500 mg of the crude tripeptide in 5 mL ethanol and 10 mL water was adjusted to pH 11-12 with 6 N NaOH in a 20 mL scintillation vial with a polycone cap. The rinsed resin was added, and gently agitated overnight in a 37° water bath. The pH was checked the next day and brought back to pH 11-12 with 6 N NaOH if needed. After another day of agitation, the resin was filtered (the peptide-containing liquid was acidified with conc HCl, evaporated and saved) and rinsed three times with 10 mL water. The unfunctionalized epoxy groups were capped by soaking the resin in 10 mL of water which contained about 0.1 mL ethanolamine for 1 hr. The resin was then rinsed three times with 10 mL water, and a sample was removed for analysis. The remainder was rinsed with 10 mL of 0.1 M NaOAc, 0.5 M NaCl pH 4.0 buffer, followed by 10 mL of 0.1 M tris chloride, 0.5 M NaCl pH 8.0 buffer. The resin was stored at 4°C in this pH 8 buffer.

### EXAMPLE 8

### Use of the Compounds of the Invention as Affinity Sorbents

A series of the compounds of formula 1 was constructed wherein YCO was γ-Glu, AA_{C} was Gly, and X was benzyl having at the para position, nitro, chloro, methoxy and methyl substituents. The relevant analogs were derivatized to Sepharose resin as described above and used as affinity sorbents in the separation of three recombinant human GST enzymes. HPLC was used to measure the relative amounts of the enzymes which bound to the supports.

The results are shown in Table 2.

**Table 2**

| Substituent | %π | %µ |
|---|---|---|
| NO₂ | 95 | 5 |
| Cl | 89 | 11 |
| OMe | 70 | 17 |
| Me | 3 | 94 |

When the percentage of π and µ isoenzymes were plotted against their sigma (meta) values good linear correlation was obtained. However, poor correlation was obtained when the sigma (para) values were used. The sigma (meta) values are a measure of purely inductive effect; however, the sigma (para) values are dependent on resonance in which the substituent can place partial charges at an atom in the ring next to the point of attachment.

The recoveries of protein and GST (CDNB-conjugating) activity in the elution fractions after affinity purification of rat liver cytosolic GST isoenzymes on various compounds of the invention are shown in Table 3.

**Table 3**

| Recovery of protein and CDNB-conjugating activity after affinity chromatography of rat liver cytosol on GSH and selected GSH analog sorbents | | | | |
|---|---|---|---|---|
| Sorbent | Protein | | Activity | |
| | (µg) | (% total) | (µmol/min/ml) | (% total) |
| HxGSH | 60 | 3.0 | 1.32 | 67 |
| S-L-GSH | 51 | 2.5 | 1.21 | 61 |
| γE-C(Cu)-G | 49 | 2.5 | 0.64 | 32 |
| γE-C(Bz)βA | 20 | 1.0 | 0.69 | 35 |
| γE-C(Bz)-A | 11 | 0.5 | 0.43 | 22 |
| γE-C(Hx)-φG | 4 | 0.2 | 0.10 | 5 |
| Abbreviation: Cu = cumyl (α,α-dimethylbenzyl) | | | | |

Traditional S-L-GSH and HxGSH sorbents showed high binding and broad isoenzyme recognition. The novel sorbents retained a lower mass of the GST isoenzymes, with the protein recovery for three of the novel sorbents being less than half that of the traditional sorbents.

In comparison with HxGSH, the novel sorbents did not show a direct correspondence of CDNB-conjugating activity with protein recovery. For example, γE-C(Bz)-βA and γE-C(Bz)-A sorbents gave 33% and 16% of the protein recovery seen with HxGSH, but the CDNB-conjugating activities were far higher (52% and 33% respectively) after correcting for the inhibitory properties of the eluting ligand. In contrast, protein recovery by γE-C(Cu)-G sorbent decreased minimally, while activity decreased by 50%. It is well known that different GST isoenzymes display individual specific activities with CDNB. The discrepancy between protein recovery and activity recovery was attributed to differences in the isoenzyme composition in the elution fractions of each sorbent. This hypothesis was supported by SDS/PAGE and reverse-phase HPLC analysis of the eluate fractions.

Fractions eluted from the sorbents listed in Table 3 were analyzed by SDS/PAGE followed by silver staining. S-L-GSH and HxGSH sorbents bound proteins which corresponded to the previously observed electrophoretic band pattern of rat liver GST subunits. The novel sorbents displayed different band patterns. For example, the γE-C(Bz)-βA eluate referred to above showed a highly enriched band for presumptive µ-class isoenzymes. As the π-class GST 7-7 is found at low levels in rat liver cytosol, other rat tissues known to express higher levels of this isoenzyme also were tested. The GST gel pattern from rat kidney cytosolic proteins eluted from the γE-C(Hx)-φG sorbent showed a protein with the same molecular migration as rat GST subunit 7 is the unique component of this eluate, indicating that this sorbent is a π-selective reagent.

A protein with a molecular migration similar to that of another GSH-dependent enzyme, glyoxalase I, was also present in the eluates from γE-C(Bz)-A and γE-C(Bz)-βA, but was not detected in eluates from the other sorbents. In these experiments HxGSH, previously shown to bind glyoxalase I, did not show any detectable levels of this GSH-dependent enzyme. Glyoxalase I is known to be a metal-dependent enzyme, and the presence of EDTA in the buffers may have interfered with binding activity.

The high degree of purity of appropriately sized proteins seen by SDS/PAGE of the affinity-eluted proteins suggested that the observed bands are indeed GSTs. Independent confirmation of this conclusion, at higher resolution, was provided by reverse-phase HPLC analyses of the GST isoenzymes in the eluates from different sorbents. Comparison with purified GST standards showed that the isoenzymes eluted from the novel sorbents correspond to known GST isoenzymes. These procedures showed that GST subunit 3 eluted at 14 min, subunit 4 at 17 min, subunit 7 at 18 min, subunit 2 at 23 min, subunit 6 at 25 min, subunits 1a and 1b at 37 and 40 min, and subunit 8 at 42 min. Subunits 3, 4 and 6 are µ-class isoenzymes, subunits 1a, 1b, 2 and 8 are α-class isoenzymes, and subunit 7 is the π-class isoenzyme. S-L-GSH and HxGSH sorbents, as expected, bound all except 5-5, the Theta-class isoenzyme.

γE-C(Cu)-G increased the proportion of isoenzyme 2, yet bound all the isoenzymes to some extent. In contrast, other sorbents were very selective in their binding of the isoenzymes. For example, γE-C(Bz)-βA selectively bound the µ-class isoenzymes 3 and 4, confirming the SDS/PAGE results.

The specificities of a variety of the novel sorbents for rat cytosolic GST isoenzymes are summarized in Table 4. Some structural alterations of the ligand produced sorbents that did not effectively bind any isoenzyme; several of these are listed for completeness.

**Table 4**

| Separation of rat GST isoenzymes by novel sorbents | | |
|---|---|---|
| Sorbent* | Isoenzyme subunit specificity | Protein recovery (%) |
| High binding, nonselective | | |
| Hx-GSH | All | 3.0 |
| S-L-GSH | All | 2.5 |
| | | |

| Selective | | |
|---|---|---|
| γE-C(Cu)-G | 2>3>4 | 2.5 |
| γE-C(BuBz)-G | 2>4>3>7 | 1.7 |
| γE-C(Bz)-A | 4>3»6>2 | 0.5 |
| γE-C(Bz)-βA | 3=4»2>6 | 1.0 |
| γE-C(Pr)-H | 4>3»2 | 1.0 |
| γE-C(Pr)-A | 3=4>2>1>6>7 | 1.4 |
| γE-C(Hx)-φG | 7»3=4 | 0.2 |
| γE-C(Bz)-φG | 7»2=3=4 | 1.3 |
| | | |

| Weak binding | | |
|---|---|---|
| γE-C(Pr)-V | Little: 2=3=4 | 0.5 |
| γE-C(Hx)-V | Little: 2-3-4 | 0.4 |
| γE-C(Pr)-D | Little: 3>2>4 | 0.2 |
| γE-C(Hx)-D | None | 0.1 |
| γE-C(Bz)-H | None | 0.1 |
| *Abbreviations as in Table 1 and text above, except Cu = cumyl (α,α-dimethylbenzyl) and φG = phenylglycine. | | |

Systematic alterations of the Cys residue to probe the previously defined H-site (hydrophobic pocket) resulted in ligands capable of binding to the isoenzymes. Adang *et al*. (1990) did not examine any analogues with systematic modifications of groups attached to the Cys moiety. Alterations of this group had effects on the level of protein recovery and the specificity of isoenzyme binding in several cases. For instance, alteration of the S-linked moiety of glutathione to a structure similar to that of cumene hydroperoxide, an α-class substrate, made the sorbents preferential for isoenzyme 2-2 of the α-class. When Ala was used as the terminal amino acid, both propyl and benzyl moieties on the Cys resulted in sorbents specific for µ-class isoenzymes (Table 4). The propyl sorbent retained more GST protein, in part because it picks up both isoenzymes 3 and 4, compared with the benzyl sorbent, which is highly specific for isoenzyme 4. When His was used as the terminal amino acid, the propyl moiety resulted in high µ-class specificity, while the benzyl moiety resulted in no binding at all. In contrast to these sorbents, alterations of the Cys moiety of Val- and Asp-substituted sorbents had no effect on the low binding. Thus, the Cys substituent can be important in modifying the overall specificity of the sorbent as determined by the terminal amino acid.

## Claims

1. An alkyl (1-6C) or arylalkyl (7-12C) ester or amide of a compound of the formula: 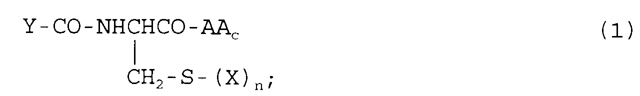
wherein: n is 1 or 2;
wherein when n is 1, X is a mono- or disubstituted or unsubstituted hydrocarbyl (1-20C) moiety optionally containing 1 or 2 nonadjacent heteroatoms (O, S or N), and wherein said substitution is selected from the group consisting of halo, -NO, -NO₂, -NR₂, OR, and SR, wherein R is H or lower alkyl (1-4C);
when n is 2, one X is as above-defined and the other X is lower alkyl (1-4C);
Y is selected from the group consisting of 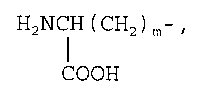 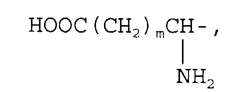 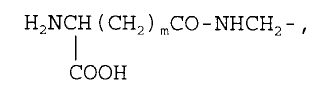 and 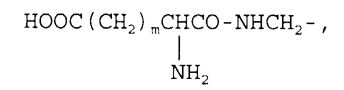
wherein m is 1 or 2; and
AA_{c} is an amino acid, coupled through a peptide bond to the remainder of the compound of formula 1, other than β-alanine or glycine.

2. The ester or amide of claim 1 wherein n is 1 and X is a substituted or unsubstituted hydrocarbyl.

3. The ester or amid of claim 2 wherein at least one X is substituted or unsubstituted methyl, propyl, hexyl, octyl, benzyl or trityl.

4. The ester or amide of any of claims 1-3 wherein AA_{c} is valine, alanine, 4-aminobutyric acid, phenylglycine, histidine, tryptophan, tyrosine, phenylalanine, or substituted phenylalanine.

5. The ester or amide of claim 4 wherein AA_{c} is alanine, histidine or phenylglycine.

6. The ester or amide of claim 5 wherein AA_{c} is phenylglycine.

7. The ester or amide of any of claims 1-6 wherein Y is

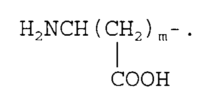

8. The ester or amide of claim 7 wherein Y-CO is γ-Glu.

9. The ester or amide of claim 8 wherein the compound of formula 1 is selected from the group consisting of γE-C(Bz)-A, γE-C(Pr)-H, γE-C(Pr)-A or γE-C(Hx)-φG.

10. The ester or amide of any of claims 1-9 that is the monoester or monoamide.

11. An alkyl (C₁₋₆) or C₇₋₁₂ aralkyl ester or amide of a compound of the formula γE-C(Bz)-φG.

12. The ester of any of claims 1-11 which is an ethyl ester or a benzyl ester.

13. The ester or amide of any of claims 1-10 that is the diester or diamide.

14. A cycloamido form of a compound of the formula 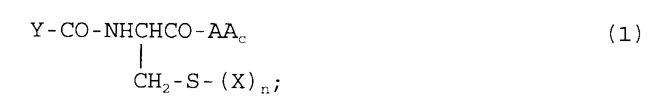 or the alkyl (1-6C) or arylalkyl (7-12C) esters or amides, or salts thereof,
wherein said cycloamido linkage is formed from an amino group of Y and a carboxyl group of AA_{c} or from a carboxyl group of Y and a free amino group of AA_{c};
wherein n is 1 or 2;
wherein when n is 1, X is H or is a mono- or disubstituted or unsubstituted hydrocarbyl (1-20C) moiety optionally containing 1 or 2 nonadjacent heteroatoms (O, S or N), and wherein said substitution is selected from the group consisting of halo, -NO, -NO₂, -NR₂, OR, and SR, wherein R is H or lower alkyl (1-4C); when n is 2, one X is as above defined and the other X is lower alkyl (1-4C);
Y is selected from the group consisting of 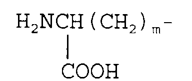 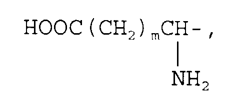 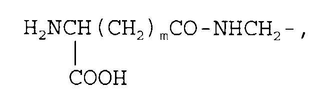 and 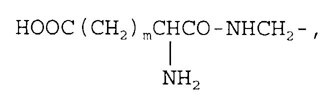
wherein m is 1 or 2; and
AA_{c} is valine, alanine, phenylglycine, histidine, tryptophan, tyrosine, phenylalanine or substituted phenlyalanine coupled through a peptide bond to the remainder of formula 1.

15. The cycloamido form of claim 14 wherein n is 1 and X is a substituted or unsubstituted hydrocarbyl.

16. The cycloamido form of claim 14 or 15 wherein Y-CO is γ-Glu.

17. The cycloamido form of any of claims 14 to 16 which is an ester or amide.

18. A compound of the formula 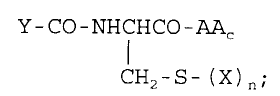 or the alkyl (1-6C) or arylalkyl (7-12C) esters or amides or salts including the cycloamido forms thereof;
wherein n is 1 or 2;
wherein when n is 1, X is a mono- di- or trisubstituted benzyl wherein said substitution is selected from the group consisting of R', halo, NO, NO₂, NH₂, OR and SR, wherein R' is alkyl (1-6C), alkenyl (1-6C) or alkynyl (1-6C), R is H or alkyl (1-6C); when n is 2, one X is as above defined and the other X is lower alkyl (1-4C);
Y is selected from the group consisting of 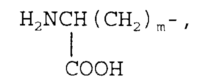 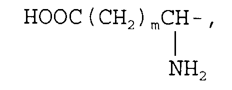 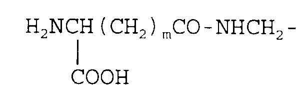 and 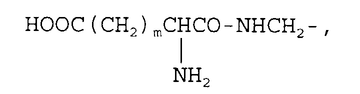 wherein m is 1 or 2; and
AA_{c} is an amino acid, coupled through a peptide bond to the remainder of the compound of formula 1, other than β-alanine or glycine;
with the proviso that if Y is 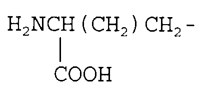 (γE), then when in the cycloamido form AA_{c} is valine, alanine, phenylglycine, histidine, tryptophan, tyrosine, phenylalanine or substituted phenylalanine.

19. The compound of claim 18 which is an ester or amide.

20. A method of purifying or characterizing a human glutathione-S-transferase (GST) enzyme from a sample, the method comprising contacting said sample with a solid support to which is coupled a compound as defined in any preceding claim under conditions wherein any human GST is adsorbed to the support,
separating the solid support from the sample; and
eluting the human GST from the solid support with an eluting solution.

21. A method according to claim 20 wherein the eluting solution contains a compound as defined in any of claims 1 to 19 with the proviso that it is not S-hexyl glutathione.

22. A method according to claim 20 or 21 wherein the sample comprises cells or tissues.

23. A method of detecting the presence or absence of a GST enzyme in a sample, the method comprising treating a sample with a compound as defined in any of claims 1 to 19 under conditions wherein a complex is formed between the GST enzyme and, if present, the compound; and
detecting the presence or absence of the complex.

24. A panel comprising at least five diverse tripeptide glutathione analogs of the formula: 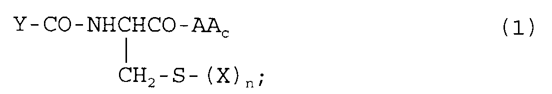 or the alkyl (1-6C) or arylalkyl (7-12C) esters or amides or salts including the cycloamido forms thereof;
wherein n is 1 or 2;
wherein when n is 1, X is H, a mono- or disubstituted or unsubstituted hydrocarbyl (1-20C) moiety optionally containing 1 or 2 nonadjacent heteroatoms (O, S or N), and wherein said substitution is selected from the group consisting of halo, -NO, -NO₂, -NR₂, OR and SR, wherein R is H or lower alkyl (1-4C); when n is 2, one X is as above defined (except H), and the other X is lower alkyl (1-4C);
Y is selected from the group consisting of 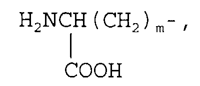 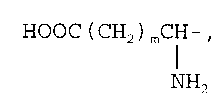 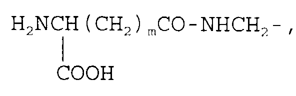 and 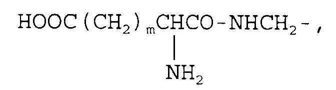 wherein m is 1 or 2; and
AA_{c} is an amino acid, coupled through a peptide bond to the remainder of the compound of formula 1, other than β-alanine or glycine;
wherein said compounds have diverse properties.

25. The panel of claim 24 wherein said diverse properties include diversity in a property selected from the group consisting of hydrophobicity of X, Hammett's Constants of X, and hydrophobicity of AA_{c}.

26. The panel of claim 24 or 25 wherein at least one of said compounds is in the cycloamido form or is an ester or amide.

27. A method to determine GST complement of a cell or tissue sample suspected of containing at least one GST enzyme, which method comprises
determining an elution characteristic with respect to each support in a panel of chromatographic supports wherein said panel comprises supports derivatized to the compounds of the panel of claim 24, 25 or 26 to obtain a survey of characteristics (SC) profile; and
comparing the resulting SC profile obtained from said tissue or cells with the reference set of SC profiles obtained from tissues or cells of known GST complements.

## Patentansprüche

1. Alkyl-(1-6C)- oder Arylalkyl-(7-12C)-ester oder -amid einer Verbindung der Formel: 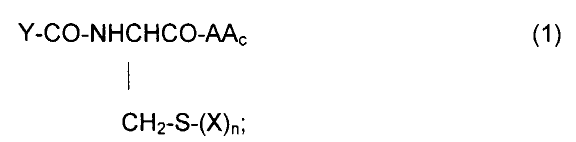
wobei: n 1 oder 2 ist;
wobei dann, wenn n 1 ist, X eine mono- oder disubstituierte oder unsubstituierte Hydrocarbyl-(1-20C)-Komponente ist, die wahlweise 1 oder 2 nicht nebeneinanderliegende Heteroatome (O, S oder N) enthält, und wobei diese Substitution gewählt ist aus der Gruppe, bestehend aus Halo, -NO, -NO₂, -NR₂, OR und SR, wobei R H oder niederes Alkyl (1-4C) ist;
wenn n 2 ist, ein X wie oben definiert ist und das andere X niederes Alkyl (1-4C) ist;
Y gewählt ist aus der Gruppe, bestehend aus 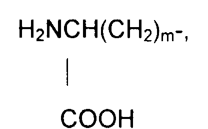 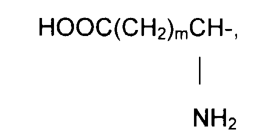 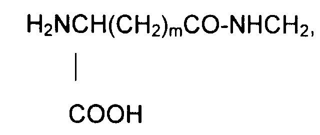 und 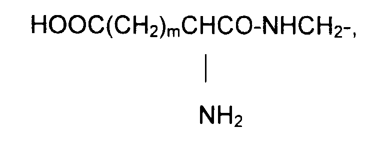 wobei m 1 oder 2 ist; und
AA_{c} eine Aminosäure ist, die durch eine Peptid-Bindung an den Rest der Verbindung der Formel 1 gekoppelt ist, außer β-Alanin oder Glycin.

2. Ester oder Amid nach Anspruch 1, wobei n 1 ist und X ein substituiertes oder unsubstituiertes Hydrocarbyl ist.

3. Ester oder Amid nach Anspruch 2, wobei mindestens ein X substituiertes oder unsubstituiertes Methyl, Propyl, Hexyl, Octyl, Benzyl oder Trityl ist.

4. Ester oder Amid nach einem der Ansprüche 1-3, wobei AA_{c} Valin, Alanin, 4-Aminobutyrsäure, Phenylglycin, Histidin, Tryptophan, Tyrosin, Phenylalanin oder substituiertes Phenylalanin ist.

5. Ester oder Amid nach Anspruch 4, wobei AA_{c} Alanin, Histidin oder Phenylglycin ist.

6. Ester oder Amid nach Anspruch 5, wobei AA_{c} Phenylglycin ist.

7. Ester oder Amid nach einem der Ansprüche 1-6, wobei Y ist

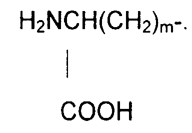

8. Ester oder Amid nach Anspruch 7, wobei Y-CO γ-Glu ist.

9. Ester oder Amid nach Anspruch 8, wobei die Verbindung der Formel 1 gewählt ist aus der Gruppe, bestehend aus γE-C(Bz)-A, γE-C(Pr)-H, γE-C(Pr)-A oder γE-C(Hx)-φG.

10. Ester oder Amid nach einem der Ansprüche 1-9, die der Monoester oder das Monoamid sind.

11. Alkyl-(C₁₋₆)- oder Arylalkyl-(C₇₋₁₂)-ester oder -amid einer Verbindung der Formel γE-C(Bz)-φG.

12. Ester nach einem der Ansprüche 1-11, welcher ein Ethylester oder ein Benzylester ist.

13. Ester oder Amid nach einem der Ansprüche 1-10, die der Diester oder das Diamid sind.

14. Eine Cycloamido-Form einer Verbindung der Formel 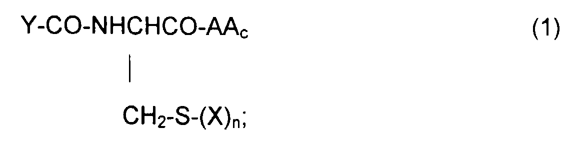 oder die Alkyl-(1-6C)- oder Arylalkyl-(7-12C)-ester oder -amide, oder deren Salze,
wobei die Cycloamido-Verknüpfung aus einer Aminogruppe von Y und einer Carboxylgruppe von AA_{c} oder aus einer Carboxylgruppe von Y und einer freien Aminogruppe von AA_{c} gebildet ist;
wobei n 1 oder 2 ist;
wobei dann, wenn n 1 ist, X H ist oder eine mono- oder disubstituierte oder unsubstituierte Hydrocarbyl-(1-20C)-Komponente, die wahlweise 1 oder 2 nicht nebeneinanderliegende Heteroatome (O, S oder N) enthält, und wobei diese Substitution gewählt ist aus der Gruppe, bestehend aus Halo, -NO, -NO₂, -NR₂, OR und SR, wobei R H ist oder niederes Alkyl (1-4C);
wenn n 2 ist, ein X wie oben definiert und das andere X niederes Alkyl (1-4C) ist;
Y gewählt ist aus der Gruppe, bestehend aus 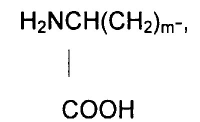 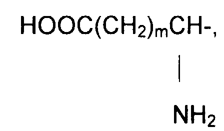 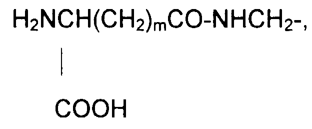 und 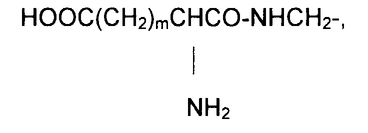 wobei m 1 oder 2 ist; und
AA_{c} Valin, Alanin, Phenylglycin, Histidin, Tryptophan, Tyrosin, Phenylalanin oder substituiertes Phenylalanin ist, das durch eine Peptid-Bindung an den Rest der Formel 1 gekoppelt ist.

15. Cycloamido-Form nach Anspruch 14, wobei n 1 und X ein substituiertes oder unsubstituiertes Hydrocarbyl ist.

16. Cycloamido-Form nach Anspruch 14 oder 15, wobei Y-CO γ-Glu ist.

17. Cycloamido-Form nach einem der Ansprüche 14 bis 16, welche ein Ester oder Amid ist.

18. Verbindung der Formel 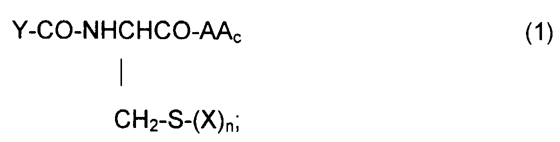 oder die Alkyl-(1-6C)- oder Arylalkyl-(7-12C)-ester oder -amide oder -salze, einschließlich deren Cycloamido-Formen;
wobei n 1 oder 2 ist;
wobei dann, wenn n 1 ist, X ein mono-, di- oder trisubstituiertes Benzyl ist, wobei diese Substitution gewählt ist aus der Gruppe, bestehend aus R', Halo, NO, NO₂, NH₂, OR und SR, wobei R' Alkyl (1-6C), Alkenyl (1-6C) oder Alkynyl (1-6C) ist, R H oder Alkyl (1-6C) ist; wenn n 2 ist, ein X wie oben definiert und das andere X niederes Alkyl (1-4C) ist;
Y gewählt ist aus der Gruppe, bestehend aus 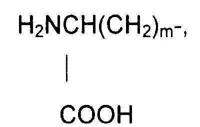 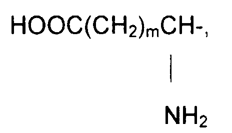 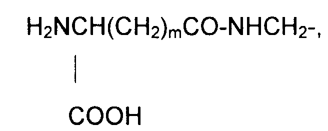 und 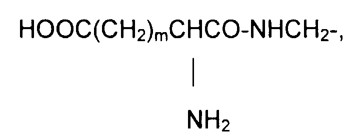 wobei m 1 oder 2 ist; und
AA_{c} eine Aminosäure ist, die durch eine Peptid-Bindung an den Rest der Verbindung der Formel 1 gekoppelt ist, außer β-Alanin oder Glycin;
unter der Voraussetzung, daß dann, wenn Y 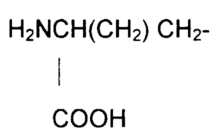 (γE) ist, AA_{c}, wenn in der Cycloamido-Form, Valin, Alanin, Phenylglycin, Histidin, Tryptophan, Tyrosin, Phenylalanin oder substituiertes Phenylalanin ist.

19. Verbindung nach Anspruch 18, welche ein Ester oder Amid ist.

20. Verfahren zur Reinigung oder Charakterisierung eines humanen Glutathion-S-Transferase-(GST)-Enzyms aus einer Probe, welches Verfahren das Zusammenbringen dieser Probe mit einer festen Trägersubstanz umfaßt, an die eine Verbindung, wie in einem der vorangehenden Ansprüche definiert, unter Bedingungen gekoppelt ist, bei denen jegliches humane GST an den Träger adsorbiert wird,
Trennen der festenTrägersubstanz von der Probe; und
Eluieren des humanen GST von der festen Trägersubstanz mit einer Elutionslösung.

21. Verfahren nach Anspruch 20, wobei die Elutionslösung eine Verbindung enthält, wie in einem der Ansprüche 1 bis 19 definiert, unter der Voraussetzung, daß sie nicht S-Hexylglutathion ist.

22. Verfahren nach Anspruch 20 oder 21, bei dem die Probe Zellen oder Gewebe umfaßt.

23. Verfahren zum Nachweis des Vorhandenseins oder der Abwesenheit eines GST-Enzyms in einer Probe, welches Verfahren die Behandlung einer Probe mit einer Verbindung, wie in einem der Ansprüche 1 bis 19 definiert, unter Bedingungen umfaßt, bei denen ein Komplex zwischen dem GST-Enzym und, falls vorhanden, der Verbindung gebildet wird; und
Nachweisen des Vorhandenseins oder der Abwesenheit des Komplexes.

24. Panel, umfassend mindestens fünf verschiedenartige Tripeptid-Glutathion-Analoga der Formel: 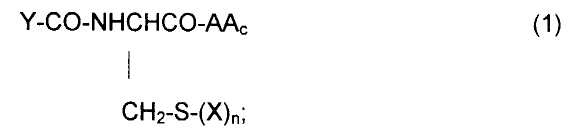 oder die Alkyl-(1-6C)- oder Arylalkyl-(7-12C)-ester oder -amide oder -salze, einschließlich deren Cycloamido-Formen;
wobei n 1 oder 2 ist;
wobei dann, wenn n 1 ist, X H ist, eine mono- oder disubstituierte oder unsubstituierte Hydrocarbyl-(1-20C)-Komponente, die wahlweise 1 oder 2 nicht nebeneinanderliegende Heteroatome (O, S oder N) enthält, und wobei diese Substitution gewählt ist aus der Gruppe, bestehend aus Halo, -NO, -NO₂, -NR₂, OR und SR, wobei R H ist oder niederes Alkyl (1-4C); wenn n 2 ist, ein X wie oben definiert ist (außer H) und das andere X niederes Alkyl (1-4C) ist;
Y gewählt ist aus der Gruppe, bestehend aus 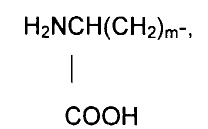 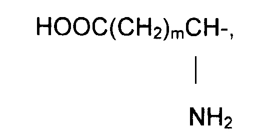 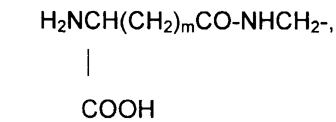 und 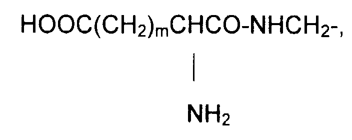 wobei m 1 oder 2 ist; und
AA_{c} eine Aminosäure ist, die durch eine Peptid-Bindung an den Rest der Verbindung der Formel 1 gekoppelt ist, außer β-Alanin oder Glycin;
wobei diese Verbindung diverse Eigenschaften aufweist.

25. Panel nach Anspruch 24, wobei diese diversen Eigenschaften die Diversität bei einer Eigenschaft umfassen, gewählt aus der Gruppe, bestehend aus Hydrophobizität von X, Hammett-Konstanten von X und Hydrophobizität von AA_{c}.

26. Panel nach Anspruch 24 oder 25, bei dem mindestens eine dieser Verbindungen in der Cycloamido-Form vorliegt oder ein Ester oder Amid ist.

27. Verfahren zur Bestimmung eines GST-Komplements einer Zell- oder Gewebsprobe, die vermutlich mindestens ein GST-Enzym enthält, welches Verfahren umfaßt:
Bestimmen einer Elution, die bezüglich jeder Trägersubstanz in einem Panel chromatographischer Trägersubstanzen charakteristisch ist, wobei das Panel Trägersubstanzen umfaßt, die zu den Verbindungen des Panels nach Anspruch 24, 25 oder 26 derivatisiert sind, um ein Profil der Charakteristiken-Erhebung (CE) zu erhalten; und
Vergleichen des resultierenden CE-Profils, das von diesem Gewebe oder Zellen erhalten wurde, mit dem Bezugsset der CE-Profile, die von Geweben oder Zellen bekannter GST-Komplemente erhalten wurden.

## Revendications

1. Un alkyle (C₁-C₆) ou arylalkyle (C₇-C₁₂) ester ou un amide d'un composé de formule : 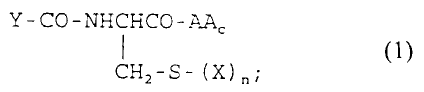
dans laquelle : n est 1 ou 2 ;
lorsque n est 1, X est une partie hydrocarbyle (C₁-C₂₀) mono- ou bisubstituée ou non substituée contenant éventuellement 1 ou 2 hétéroatomes (O, S ou N) non adjacents, et où ladite substitution est choisie dans le groupe constitué par halo, -NO, -NO₂, -NR₂, OR et SR, où R est H ou un alkyle inférieur (C₁-C₄) ;
lorsque n est 2, un des X est tel que défini ci-dessus et l'autre X est un alkyle inférieur (C₁-C₄) ;
Y est choisi dans le groupe constitué par 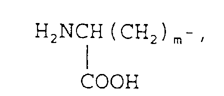 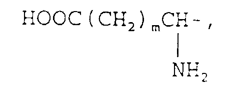 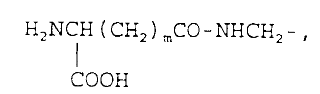 et 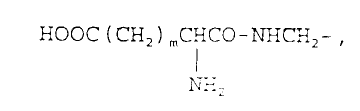 où m est 1 ou 2 ; et
AA_{c} est un acide aminé, couplé via une liaison peptidique au reste du composé de formule 1, autre que la β-alanine ou la glycine.

2. Ester ou amide selon la revendication 1 dans lequel n est 1 et X est un hydrocarbyle substitué ou non substitué.

3. Ester ou amide selon la revendication 2 dans lequel au moins un des X est un méthyle, propyle, hexyle, octyle, benzyle ou trityle substitué ou non substitué.

4. Ester ou amide selon l'une quelconque des revendications 1 à 3 dans lequel AA_{c} est la valine, l'alanine, l'acide 4-aminobutyrique, la phénylglycine, l'histidine, le tryptophane, la tyrosine, la phénylalanine, ou la phénylalanine substituée.

5. Ester ou amide selon la revendication 4 dans lequel AA_{c} est l'alanine, l'histidine ou la phénylglycine.

6. Ester ou amide selon la revendication 5 dans lequel AA_{c} est la phénylglycine.

7. Ester ou amide selon l'une quelconque des revendications 1 à 6 dans lequel Y est

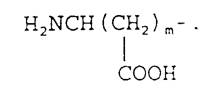

8. Ester ou amide selon la revendication 7 dans lequel Y-CO est γ-Glu.

9. Ester ou amide selon la revendication 8 dans lequel le composé de formule 1 est choisi dans le groupe constitué par γE-C(Bz)-A, γE-C(Pr)-H, γE-C(Pr)-A ou γE-C(Hx)-φG.

10. Ester ou amide selon l'une quelconque des revendications 1 à 9 qui est le monoester ou le monoamide.

11. Alkyle (C₁₋₆) ou aralkyle (C₇₋₁₂) ester ou amide d'un composé de formule γE-C(Bz)-φG.

12. Ester selon l'une quelconque des revendications 1 à 11 qui est un ester éthylique ou un ester benzylique.

13. Ester ou amide selon l'une quelconque des revendications 1 à 10 qui est un diester ou un diamide.

14. Une forme cycloamido d'un composé de la formule 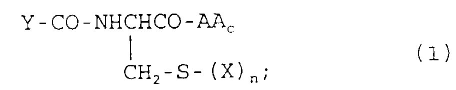 ou les alkyl (C₁-C₆) ou arylalkyl (C₇-C₁₂) esters ou amides, ou leurs sels,
dans laquelle ladite liaison cycloamido est formée à partir d'un groupe amino de Y et d'un groupe carboxyle de AA_{c} ou à partir d'un groupe carboxyle de Y et d'un groupe amino libre de AA_{c} ;
dans laquelle n est 1 ou 2 ;
lorsque n est 1, X est H ou est une partie hydrocarbyle (C₁-C₂₀) mono- ou bisubstituée ou non substituée contenant éventuellement 1 ou 2 hétéroatomes (O, S ou N) non adjacents, et où ladite substitution est choisie dans le groupe constitué par halo, -NO, -NO₂, -NR₂, OR et SR, où R est H ou un alkyle inférieur (C₁-C₄) ;
lorsque n est 2, un X est tel que défini ci-dessus et l'autre X est un alkyle inférieur (C₁-C₄) ;
Y est choisi dans le groupe constitué par 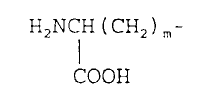 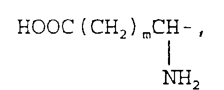 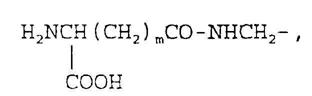 et 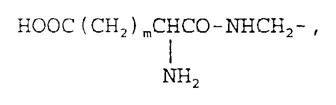 où m est 1 ou 2 ; et
AA_{c} est la valine, l'alanine, la phénylglycine, l'histidine, le tryptophane, la tyrosine, la phénylalanine ou la phénylalanine substituée couplé via une liaison peptidique au reste de la formule 1.

15. La forme cycloamido selon la revendication 14 dans laquelle n est 1 et X est un hydrocarbyle substitué ou non substitué.

16. La forme cycloamido selon la revendication 14 ou 15 dans laquelle Y-CO est γ-Glu.

17. La forme cycloamido selon l'une quelconque des revendications 14 à 16 qui est un ester ou un amide.

18. Composé de formule 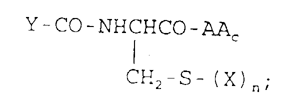 ou les alkyl (C₁-C₆) ou arylalkyl (C₇-C₁₂) esters ou amides ou les sels comprenant les formes cycloamido de ceux-ci ;
dans lequel n est 1 ou 2 ;
lorsque n est 1, X est un benzyle mono-, bi- ou trisubstitué où ladite substitution est choisie dans le groupe constitué par R', halo, NO, NO₂, NH₂, OR et SR, où R' est un alkyle (C₁-C₆), alkényle (C₁-C₆) ou alkynyle (C₁-C₆), R est H ou un alkyle (C₁-C₆) ;
lorsque n est 2, un des X est tel que défini ci-dessus et l'autre X est un alkyle inférieur (1-4C) ;
Y est choisi dans le groupe constitué par 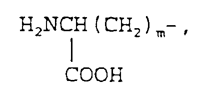 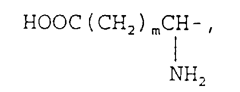 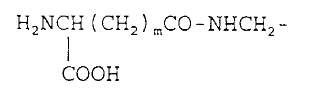 et 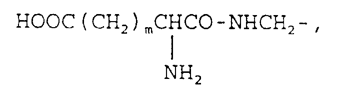 où m est 1 ou 2 ; et
AA_{c} est un acide aminé, couplé via une liaison peptidique au reste du composé de formule 1, autre que la β-alanine ou la glycine ;
sous réserve que lorsque Y est 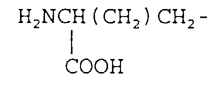 (γE) alors, lorsqu'il est sous la forme cycloamido, AA_{c} est la valine, l'alanine, la phénylglycine, l'histidine, le tryptophane, la tyrosine, la phénylalanine ou la phénylalanine substituée.

19. Composé selon la revendication 18 qui est un ester ou un amide.

20. Méthode de purification ou de caractérisation d'une enzyme glutathion-S-transférase (GST) humaine à partir d'un échantillon, la méthode comprenant la mise en contact dudit échantillon avec un support solide auquel est couplé un composé tel que défini selon l'une quelconque des revendications précédentes dans des conditions dans lesquelles toute GST humaine est adsorbée au support,
la séparation du support solide de l'échantillon ; et
l'élution de la GST humaine à partir du support solide avec une solution d'élution.

21. Méthode selon la revendication 20 dans laquelle la solution d'élution contient un composé tel que défini selon l'une quelconque des revendications 1 à 19 à condition qu'il ne soit pas le S-hexyl-glutathion.

22. Méthode selon la revendication 20 ou 21 dans laquelle l'échantillon comprend des cellules ou des tissus.

23. Méthode de détection de la présence ou de l'absence d'une enzyme GST dans un échantillon, la méthode comprenant le traitement d'un échantillon avec un composé tel que défini selon l'une quelconque des revendications 1 à 19 dans des conditions dans lesquelles un complexe est formé entre l'enzyme GST et, s'il est présent, le composé ; et
la détection de la présence ou de l'absence du complexe.

24. Ensemble comprenant au moins cinq analogues tripeptidiques de glutathion différents de formule : 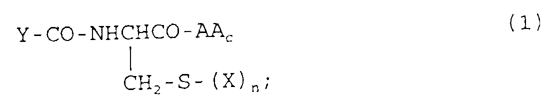 ou les alkyl (C₁-C₆) ou arylalkyl (C₇-C₁₂) esters ou amides ou les sels comprenant les formes cycloamido de ceux-ci ;
dans lequel n est 1 ou 2 ;
lorsque n est 1, X est H, une partie hydrocarbyle (C₁-C₂₀) mono- ou bisubstituée ou non substituée contenant éventuellement 1 ou 2 hétéroatomes (O, S ou N) non adjacents, et où ladite substitution est choisie dans le groupe consittué par halo, -NO, -NO₂, -NR₂, OR et SR, où R est H ou un alkyle inférieur (C₁-C₄) ;
lorsque n est 2, un des X est tel que défini ci-dessus (excepté H), et l'autre X est un alkyle inférieur (1-4C) ;
Y est choisi dans le groupe constitué par 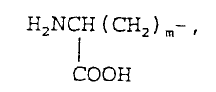 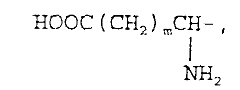 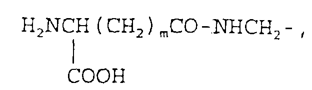 et 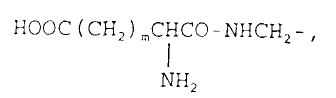 où m est 1 ou 2 ; et
AA_{c} est un acide aminé, couplé via une liaison peptidique au reste du composé de formule 1, autre que la β-alanine ou la glycine ;
ensemble dans lequel lesdits composés ont diverses propriétés.

25. Ensemble selon la revendication 24 dans lequel lesdites diverses propriétés comprennent une diversité dans une propriété choisie dans le groupe constitué par l'hydrophobicité de l'X, la constante de Hammett de X, et l'hydrophobicité de AA_{c}.

26. Ensemble selon la revendication 24 ou 25 dans lequel au moins un desdits composés est sous la forme cycloamido ou est un ester ou un amide.

27. Méthode pour déterminer le complément d'une GST d'un échantillon de cellules ou de tissu soupçonné de contenir au moins une enzyme GST, méthode comprenant
la détermination d'une caractéristique d'élution par rapport à chacun des supports dans un ensemble de supports chromatographiques où ledit ensemble comprend des supports greffés à des composés de l'ensemble de la revendication 24, 25 ou 26 pour obtenir un aperçu du profil de caractéristiques (SC) ; et
la comparaison du profil SC résultant obtenu à partir dudit tissu ou desdites cellules à l'ensemble de référence des profils SC obtenus à partir des tissus ou cellules de compléments connus de GST.
